# EUROPEAN PATENT APPLICATION

(11) **EP 3 818 983 A1**
(43) Date of publication of application: **12.05.2021**
(21) Application number: 19382990.0
(22) Date of filing: 11.11.2019
(51) Int. Cl.: A61K 31/6615, A61K 45/06, A61P 9/10, A61P 9/14

(54) **INOSITOL PHOSPHATE COMPOUNDS FOR USE IN TREATING, INHIBITING THE PROGRESSION, OR PREVENTING CARDIOVASCULAR CALCIFICATION**

(71) Applicant: Sanifit Therapeutics S.A., 07121 Palma de Mallorca (Islas Baleares) (ES)
(72) Inventor: PERELLÓ BESTARD, Joan, 07121 Palma de Mallorca (Islas Baleares) (ES); SALCEDO ROCA, Carolina, 07121 Palma de Mallorca (Islas Baleares) (ES); FERRER REYNÉS, Miquel David, 07121 Palma de Mallorca (Islas Baleares) (ES)
(74) Representative: Pons

(57) **Abstract**

The present invention relates to compounds, pharmaceutical compositions, combined preparations, and dosage regimens for treating, inhibiting the progression, and preventing cardiovascular calcification, and in particular, coronary calcification, aortic artery calcification, and aortic valve calcification comprising inositol phosphates. In a particular aspect the disclosure provides a dosage regimen for treating, inhibiting the progression, or preventing cardiovascular calcification comprising the administration of about 200 mg to about 700 mg of myo-inositol hexaphosphate per administration.

## Description

### Field of the Invention

The present invention relates to the use of inositol phosphates (IP), their analogs and derivatives for treating, inhibiting the progression, or preventing cardiovascular calcification in human health. The present invention also relates to pharmaceutical compositions and combined preparations comprising said IP and a dosage regime for their administration.

### Background Art

The relative risk of death due to cardiovascular causes is 5 to 30 times higher in patients with end-stage kidney disease (ESKD) requiring dialysis than in the general population (Foley R, et al., Am J Kidney Dis. 1998; 32:S112-119). Although traditional risk factors such as diabetes mellitus and hypertension are highly prevalent in patients with chronic kidney disease (CKD), they account for only a portion of the increased cardiovascular risk (Cozzolino M, et al., Nephrol Dial Transplant. 2018; 33:iii28-iii34). In clinical practice, at least 80% of patients who receive maintenance hemodialysis have evidence of cardiovascular calcification (Raggi P, et al., J Am Coll Cardiol. 2002;39: 695-701 and Bellasi A, et al., Kidney Int. 2006; 70:1623-1628). This marker of vasculopathy has been linked with morbidity and mortality in the general population and in patients receiving maintenance hemodialysis (Blaha M, et al., Lancet 2011; 378:684-6 and Chen J, et al., JAMA Cardiol. 2017; 2:635-643).

In patients with advanced CKD, cardiovascular calcification is likely secondary to a combination of accelerated atherosclerosis and, perhaps more so, to arteriosclerosis related to derangements of mineral metabolism (Johnson R, et al., Circ Res. 2006; 99:1044-1059 and Raggi P, et al., Nat Clin Pract Cardiovasc Med. 2007; 4:26-33). Cardiovascular calcification is a highly regulated process that resembles bone formation and ultimately depends upon HAP nucleation and crystal growth. Patients with CKD and cardiovascular calcification have increased morbidity and mortality through several pathways, including coronary atherosclerosis, arterial stiffness, left ventricular hypertrophy, myocardial ischemia, and electrocardiographic abnormalities. (Brown A, et al., Nat Rev Cardiol. 2016; 13:210-220, Raggi P, et al., Kidney Int. 2007;71:802-807, Di lorio B, et al., Kidney Blood Press Res. 2011; 34:180-187, Kitamura K, et al., Heart Vessels. 2017; 32:1109-1116, and Karohl C, et al., J Nucl Cardiol. 2013; 20:1013-1020).

There is evidence that attenuating the progression of vascular calcification in CKD is associated with a reduction in mortality (Jamal S, et al., Lancet 2013; 382:1268-1277). The phosphate binder sevelamer and the calcimimetic cinacalcet used to treat hyperphosphatemia and secondary hyperparathyroidism, respectively, may reduce progression of vascular calcification in ESKD. However, these drugs do not fully address the complex biology responsible for vascular calcification (Patel L, et al., Clin J Am Soc Nephrol. 2016; 11:232-244, and Raggi P, et al., Nephrol Dial Transplant. 2011; 26:1327-1339.

SNF472, a formulation of myo-inositol hexaphosphate, acts through a novel pathway to selectively and directly inhibit the formation and growth of HAP crystals, the final common step in the pathophysiology of vascular calcification (Ferrer M, et al., Sci Rep. 2017;7:6858 and Ferrer M, et al., PLoS ONE 2018; 13:e0197061). Infusing SNF472 during each dialysis session achieves therapeutic levels, ensuring treatment adherence without additional burden to the patient (Perelló J, et al., J Nephrol. 2018;31:287-296). A phase 1 clinical trial showed that in patients receiving maintenance hemodialysis, administration of a single dose of SNF472 at 9 mg/kg inhibited HAP crystallization potential by 80%, compared to 9% with placebo (Perelló J, et al., Br J Clin Pharmacol. 2018; 84:2867-2876.). Thus, SNF472 could be candidate for attenuating the progression of cardiovascular calcification in dialyzed patients.

### Summary of the Invention

In a first aspect, the present invention relates to a compound of general formula I, or a pharmaceutically acceptable salt thereof: 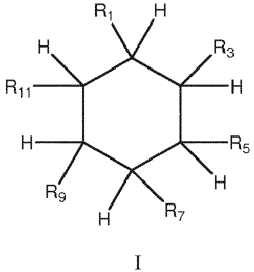 wherein:
(i) R₁, R₃, R₅, R₇, R₉, and R₁₁ are independently selected from OH, a radical of formula II, III, IV and a heterologous moiety: 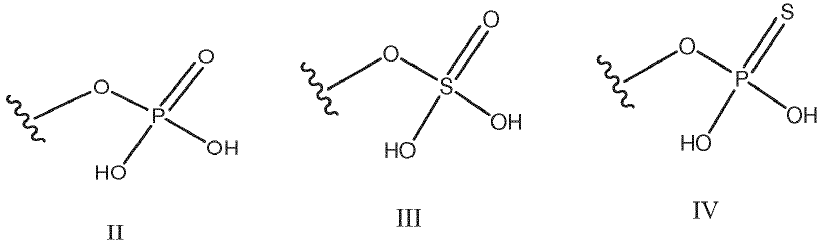
(ii) at least one of R₁, R₃, R₅, R₇, R₉, and R₁₁ is selected from a radical of formula II, III and IV; and
(iii) zero, one, two or three of R₁, R₃, R₅, R₇, R₉, and R₁₁ is a heterologous moiety;
for use in the treatment, inhibition of progression, or prevention of cardiovascular calcification, or a disease, condition or symptom associated with cardiovascular calcification in a subject in need thereof, wherein (a) the compound is in a form suitable for parenteral, topical, or enteral administration, and (b) the compound is administered to the subject in a non-bolus prolonged release form in an effective dosage of about 200 mg to about 700 mg per administration.

In some aspects, the present invention refers to a compound of general formula I, as defined above, wherein the heterologous moiety is selected from a radical of formula V, a radical of formula VI, and a radical of formula VII: 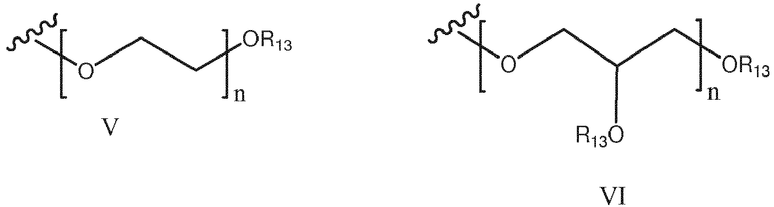 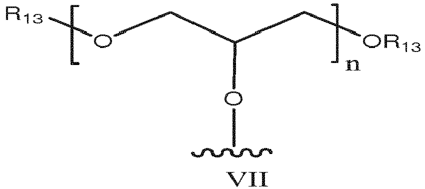 and wherein n is an integer in the range from 2 to 200, and R₁₃ is selected from H, methyl, or ethyl.

Alternatively, the invention refers to a compound of general formula I, as defined above, for use in the treatment, inhibition of progression, or prevention of cardiovascular calcification in a subject in need thereof, wherein (a) the compound is in a form suitable for parenteral, topical, or enteral administration, and (b) the compound is administered to the subject in a non-bolus prolonged release form in a dosage of about 200 mg to about 700 mg per administration, and (c) the administration of the compound treats, inhibits the progression, or prevents cardiovascular calcification, or a disease, condition or symptom associated with cardiovascular calcification in the subject.

In a further aspect, the invention also relates to a method for treating, inhibiting the progression, or preventing cardiovascular calcification which comprises administering a therapeutically effective amount of a compound of formula I, as defined above, together with pharmaceutically acceptable excipients or carriers, to a subject in need thereof. This aspect may also be formulated as the use of a compound of formula I, as defined above, for the manufacture of a medicament for treating, inhibiting the progression, or preventing cardiovascular calcification in a subject in need thereof.

In another aspect, the invention also relates to a method for treating, inhibiting the progression, or preventing coronary artery calcification which comprises administering a therapeutically effective amount of a compound of formula I, as defined above, together with pharmaceutically acceptable excipients or carriers, to a subject in need thereof. This aspect may also be formulated as the use of a compound of formula I, as defined above, for the manufacture of a medicament for treating, inhibiting the progression, or preventing coronary artery calcification in a subject in need thereof.

In an additional aspect, the invention relates to a method for treating, inhibiting the progression, or preventing aortic artery calcification which comprises administering a therapeutically effective amount of a compound of formula I, as defined above, together with pharmaceutically acceptable excipients or carriers, to a subject in need thereof. This aspect may also be formulated as the use of a compound of formula I, as defined above, for the manufacture of a medicament for treating, inhibiting the progression, or preventing aortic artery calcification in a subject in need thereof.

In a further aspect, the invention relates to a method for treating, inhibiting the progression, or preventing aortic valve calcification which comprises administering a therapeutically effective amount of a compound of formula I, as defined above, together with pharmaceutically acceptable excipients or carriers, to a subject in need thereof. This aspect may also be formulated as the use of a compound of formula I, as defined above, for the manufacture of a medicament for treating, inhibiting the progression, or preventing aortic valve calcification in a subject in need thereof.

The compounds of the present invention are particularly useful for treating, inhibiting the progression, or preventing coronary artery calcification, aortic artery calcification and/or aortic valve calcification in dialyzed patients, and in particular, in dialyzed patients with kidney failure.

The invention also provides a pharmaceutical composition comprising at least one compound of formula I, as defined above, for use in: (i) treating, inhibiting the progression, or preventing cardiovascular calcification, (ii) for treating, inhibiting the progression, or preventing coronary artery calcification, (iii) for treating, inhibiting the progression, or preventing aortic artery calcification and/or (iii) or treating, inhibiting the progression, or preventing aortic valve calcification in a subject in need thereof.

In an additional aspect, the invention refers to a combined preparation comprising at least a compound of formula I or a pharmaceutical composition according to the invention and at least a second active agent and the use of said combined preparation in human health, and in particular, in dialyzed patients with kidney failure.

### Brief Description of the Drawings

**Fig. 1** shows representative inositol phosphate analogs in which two out of six X are OPSO₂²⁻ and the remaining X are OSO₃. Four specific forms of 4,6-di-(O-thiophosphate)-inositol-1 ,2,3,5-tetra-O-sulfate are shown.
**Fig. 2** shows inositol phosphate analogs and inositol phosphate derivatives that can be used to practice the methods of the present invention. The molecules shown are myo-inositol-pentakisphosphate-2-PEG400, myo-inositol hexakissulfate (myo-inositol hexasulfate), and scyllo-myo-inositol hexakissulfate (scyllo-inositol hexasulfate).
**Fig. 3** shows inositol phosphate analogs and inositol phosphate derivatives that can be used to practice the methods of the present invention. X represent independently phosphorus and/or sulfur containing groups (e.g., phosphate, sulfate, or thiophosphate). R¹ represents a heterologous moiety (e.g., PEG or PG).
**Fig. 4** shows exemplary inositol phosphate analogs and inositol phosphate derivatives that can be used to practice the methods of the present invention. R¹ represents a heterologous moiety (e.g., PEG or PG). n can be between 2 and 200.
**Fig. 5** shows exemplary inositol phosphate analogs and inositol phosphate derivatives that can be used to practice the methods of the present invention. n can be between 2 and 200.
**Fig. 6** shows exemplary inositol phosphate analogs and inositol phosphate derivatives that can be used to practice the methods of the present invention. n can be between 2 and 200.
**Fig. 7** illustrates the patient disposition of the clinical study. 231 patients did not have a coronary artery calcium score in the required range at screening (*). Other reasons for excluding patients included that the computed tomography scan was not completed or evaluable, kidney transplantation, or that the screening/enrollment has been closed (†).
**Fig. 8** shows the mean (95% CI) change from baseline to week 52 for calcium scores in the SNF472 combined dosing groups versus placebo. Modified intention-to-treat (mITT) population with last observation carried forward (LOCF). A, Coronary artery calcium volume. B, Coronary artery Agatston score. C, Aortic valve calcium volume. D, Aortic valve Agatston score. E, Thoracic aorta calcium volume. F, Thoracic aorta Agatston score.
**Fig. 9** shows the progression of calcium volume and Agatston scores obtained in the per-protocol (PP) population of patients that completed 52 weeks of treatment. A, Coronary artery calcium volume. B, Coronary artery Agatston score.
**Fig. 10** shows the proportion of patients with < 15% progression in CAC Agatston score at week 52. A, in the mITT population. B, in the PP population.
**Fig. 11** shows the mean (95% CI) change from baseline to week 52 for calcium scores in the SNF472 combined dosing groups and individual dosing groups versus placebo.
Modified intention-to-treat population (mITT) with last observation carried forward (LOCF). A, Coronary artery calcium volume. B, Coronary artery Agatston score. C,
Aortic valve calcium volume. D, Aortic valve Agatston score. E, Thoracic aorta calcium volume. F, Thoracic aorta Agatston score.
**Fig. 12** shows the mean the sensitivity analyses of mean (95% CI) change from baseline to week 52 for calcium scores in the SNF472 combined dosing groups versus placebo. Modified intention-to-treat (mITT) population with multiple imputation methods. A, Coronary artery calcium volume score. B, Coronary artery calcium Agatston score. C, Aortic valve calcium volume score. D, Aortic valve calcium Agatston score. E, Thoracic aorta calcium volume score. F, Thoracic aorta calcium Agatston score.

### Detailed Description of the Invention

The present invention provides compounds, pharmaceutical compositions, combined preparations, methods and routes of administration for use in treating, inhibiting the progression, or preventing cardiovascular calcification. The invention also provides compounds, pharmaceutical compositions, methods and routes of administration for use in treating, inhibiting the progression, or preventing coronary artery calcification, aortic artery calcification and/or aortic valve calcification.

The compounds, pharmaceutical compositions, combined preparations, methods and routes of administration of the present invention are particularly useful for treating, inhibiting the progression, or preventing coronary artery calcification, aortic artery calcification and/or aortic valve calcification in dialyzed patients, and more specifically, in dialyzed patients with kidney failure.

### Definitions of general terms and expressions

The present invention includes embodiments in which exactly one member of the group is present in, employed in, or otherwise relevant to a given product or process. It also includes embodiments in which more than one, or all of the group members are present in, employed in, or otherwise relevant to a given product or process.

Unless defined otherwise, all technical and scientific terms used herein have the same meaning as commonly understood by a skilled person in the art to which this description is related. For example, the Pei-Show J, Concise Dictionary of Biomedicine and Molecular Biology, 2nd Ed. (CRC Press, Boca Raton, FL, USA 2002); Lackie J, The Dictionary of Cell and Molecular Biology, 5th Ed. (Academic Press, Cambridge, MA, USA 2013) and Cammack R, et al., Oxford Dictionary of Biochemistry and Molecular Biology, 2nd Ed., (Oxford University Press, Oxford, GB, 2006) provide the skilled person with a general dictionary of many of the terms used in this description.

Units, prefixes, and symbols are denoted in their Système International de Unites (SI) accepted form. Numeric ranges are inclusive of the numbers defining the range. Where a range of values is recited, it is to be understood that each intervening integer value, and each fraction thereof, between the recited upper and lower limits of that range is also specifically disclosed, along with each subrange between such values. The upper and lower limits of any range can independently be included in or excluded from the range, and each range where either, neither or both limits are included is also encompassed within the invention.

Where a value is explicitly recited, it is to be understood that values which are about the same quantity or amount as the recited value are also within the scope of the invention. Where a combination is disclosed, each subcombination of the elements of that combination is also specifically disclosed and is within the scope of the invention. Conversely, where different elements or groups of elements are individually disclosed, combinations thereof are also disclosed. Where any element of an invention is disclosed as having a plurality of alternatives, examples of that invention in which each alternative is excluded singly or in any combination with the other alternatives are also hereby disclosed; more than one element of an invention can have such exclusions, and all combinations of elements having such exclusions are hereby disclosed.

The term "and/or" as used herein is to be taken as the specific disclosure of each of the two specified features or components with or without the other. Thus, the term "and/or" as used in a phrase such as "A and/or B" herein is intended to include "A and B", "A or B", "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

The term "aortic artery calcification", as used herein, refers to the formation of calcium deposits in the aortic artery. Types of aortic artery calcification include, but are not limited to the calcification of the abdominal and thoracic aortic arteries and the common iliac external and internal arteries (e.g., femoral, lateral sacral).

The term "aortic valve calcification", as used herein, refers to the formation of calcium deposits in the aortic valve. The calcification of the aortic valve may result in the narrowing of the aortic valve, a condition defined as aortic valve stenosis or "AVS". Blood flow from the heart may be reduced due to AVS and cause the overexertion and weakness of the heart muscle.

The term "approximately" as used herein and as applied to one or more values of interest, refers to a value that is similar to a stated reference value. In certain aspects, the term "approximately" refers to a range of values that fall within 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

The term "cardiovascular calcification" as used herein, and its related term "CVC", refer to the formation of calcium deposits in cardiac blood vessels. The calcification can occur in the intimal (inside) or medial (middle layer) part of the blood vessel. Types of cardiovascular calcification include, but are not limited to coronary artery calcification, aortic artery calcification, and aortic valve calcification. Diseases, conditions and/or symptoms associated to cardiovascular calcification (e.g. coronary artery calcification, aortic artery calcification, aortic valve calcification) include, but are not limited to, angina pectoris, aneurysm, atherosclerosis (e.g., coronary atherosclerosis), arterial stiffness, arteriosclerosis, cardiac disease, cerebrovascular disease, coronary disease, electrocardiographic abnormalities, heart failure, congestive heart failure, hypertension, left ventricular hypertrophy, myocardial infarction, myocardial ischemia, peripheral artery disease, peripheral vascular disease, and thrombosis.

The term "compound" as used herein is meant to include all isomers and isotopes of the structure depicted. As used herein, the term "isomer" means any geometric isomer, tautomer, zwitterion, stereoisomer, enantiomer, or diastereomer of a compound. Compounds can include one or more chiral centers and/or double bonds and can thus exist as stereoisomers, such as double-bond isomers (i.e., geometric E/Z isomers) or diastereomers (e.g., enantiomers (i.e., (+) or (-)) or cis/trans isomers). The present invention encompasses any and all isomers of the compounds described herein, including stereomerically pure forms (e.g., geometrically pure, enantiomerically pure, or diastereomerically pure) and enantiomeric and stereoisomeric mixtures, e.g., racemates. Enantiomeric and stereomeric mixtures of compounds and means of resolving them into their component enantiomers or stereoisomers are well-known. A compound, salt, or complex of the present invention can be prepared in combination with solvent or water molecules to form solvates and hydrates by routine methods.

The term "coronary artery calcification" as used herein refers to refers to the formation of calcium deposits in the coronary artery.

The term "effective amount" as used herein, in reference to (i) a compound of a general formula I (e.g. an inositol phosphate, an inositol phosphate analog, an inositol phosphate derivative, or a combination thereof), or (ii) a pharmaceutical composition comprising at least one of the item (i) compounds, is that amount sufficient to effect beneficial or desired results. In some embodiments, the beneficial or desired results are, for example, clinical results, and, as such, an "effective amount" depends upon the context in which it is being applied. In the context of administering an active agent that prevents, inhibits the progression, or treats cardiovascular calcification, an effective amount of an active agent is, for example, an amount sufficient for (a) inhibiting the formation and/or growth of HAP crystals in a specific area of cardiovascular tissue in a subject, (b) slowing the progression of formation and/or growth of HAP crystals in a specific area of cardiovascular tissue in a subject, or (c) reducing, stopping or eliminating the symptoms associated to cardiovascular calcification in a subject in need thereof, as compared to the same parameters observed in the subject before the administration of the active agent, or in a population of control subjects without administration of the active agent.

The term "kidney failure" as used herein, refers to a disease that causes a progressive loss of kidney function, with a concomitant decrease in the glomerular filtration rate (GFR) or index. Kidney failure is also known as renal impairment or kidney disease. Kidney disease can be classified as (i) acute kidney injury (AKI), a progressive loss of kidney function, which generally causes oliguria and a fluid and electrolyte imbalance and (ii) chronic kidney disease (CKD), a much slower loss of kidney function over a period of months or years. Depending on the degree of kidney function, five stages of CKD are defined on the basis of the GFR: (a) stage 1, normal or high GFR (>90 mL/min), (b) stage 2: Mild CKD, GFR=60-89 mL/min, (c) stage 3, moderate CKD, GFR=30-59 mL/min, (d) stage 4, severe CKD, GFR=15-29 mL/min and (e) stage 5, terminal CKD, GFR<15 mL/min. In stage 5, dialysis or a kidney transplant are required to maintain the state of health. AKI and CKD may occur concomitantly, which is known as acute-on-chronic renal failure.

The term "excipient" as used herein, refers to a substance which helps absorption of the elements of the pharmaceutical composition, stabilizes said elements, activates or helps preparation of the composition. Thus, examples of excipients used in parenteral formulations include, but are not limited to, antimicrobial agents (e.g., benzalkonium chloride, metacresol, thimerosal), co-solvents (e.g., ethanol), buffers and pH adjusting factors (e.g., carbonate, citrate, phosphate solutions).

The terms "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and, intrasternal injection and intravenous infusion.

The term "pharmaceutically acceptable vehicle" as used herein, refers to a substance used in a composition for diluting any of the compounds, excipients or components contained therein to a determined volume or weight. The pharmaceutically acceptable vehicle is an inert substance or a substance with an analogous action to any of the elements comprising the pharmaceutical composition of the present invention. The role of said vehicle is to allow the incorporation of other elements, allow better dosing and administration or to provide consistency and shape to the composition.

The terms "prevent", "preventing" and "prevention" as used herein refer to inhibiting the inception or decreasing the occurrence of a disease, condition or symptom associated to cardiovascular calcification in a subject (e.g., preventing the formation or growth of HAP crystals in coronary arteries, aorta or aortic valves).

The term "SNF472" as used herein refers to a myo-inositol hexaphosphate hexasodium formulation. SNF472 is manufactured by dissolving myo-inositol hexaphosphate hexasodium in saline solution, followed by pH adjustment and aseptic filtration. SNF472 is prepared at three different strengths: (a) (i) 20 mg/mL and (ii) 90 mg/mL in 5 mL single-use vials, formulated in saline solution, pH 5.8 to 6.2 and (b) 30 mg/L in 10 mL single-use vials, formulated in saline solution, pH 5.6 to 6.4.

The terms "subject", "individual", "animal" or "mammal" as used herein is meant any subject, particularly a mammalian subject, for whom diagnosis, prognosis, or therapy is desired. Mammalian subjects include, but are not limited to, humans, domestic animals, farm animals, zoo animals, sport animals, pet animals such as dogs, cats, guinea pigs, rabbits, rats, mice, horses, cattle, cows; primates such as apes, monkeys, orangutans, and chimpanzees; canids such as dogs and wolves; felids such as cats, lions, and tigers; equids such as horses, donkeys, and zebras; bears, food animals such as cows, pigs, and sheep; ungulates such as deer and giraffes; rodents such as mice, rats, hamsters and guinea pigs; and so on. In certain aspects, the subject is a human subject. In some aspects, the subject is a human patient with cardiovascular calcification, or having a disease, condition or symptom associated with cardiovascular calcification, or at risk of developing said cardiovascular calcification or associated disease, condition or symptom. In some further aspects, the subject is a human patient with coronary artery calcification, aortic artery calcification, or aortic valve calcification, or having a disease, condition or symptom associated to coronary artery calcification, aortic artery calcification, or aortic valve calcification, or at risk of developing coronary artery calcification, aortic artery calcification, or aortic valve calcification or a disease, condition or symptom associated to any of them.

The term "substantially" as used herein refers to the qualitative condition of exhibiting total or near-total extent or degree of a characteristic or property of interest. A person skilled in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena.

The terms "treat" or "treatment" as used herein refer to the administration of compound or pharmaceutical composition of the present invention for (i) slowing, (ii) inhibiting the progression, (iii) stopping, or (iv) reverting the progression of a disease or condition after its clinical signs have appeared. Control of the disease progression is understood to mean the beneficial or desired clinical results that include, but are not limited to, reduction of the symptoms, reduction of the duration of the disease, stabilization of pathological states (specifically to avoid additional deterioration), delaying the progression of the disease, improving the pathological state and remission (both partial and total). The control of progression of the disease also involves an extension of survival compared with the expected survival if treatment was not applied. Within the context of the present invention, the terms "treat" and "treatment" refer specifically to (a) inhibiting the further formation and/or growth of HAP crystals in a specific area of cardiovascular tissue in a subject, (b) slowing the progression of formation and/or growth of HAP crystals in a specific area of cardiovascular tissue in a subject, or (c) reducing, stopping or eliminating the symptoms associated to cardiovascular calcification in a subject in need thereof.

### Compounds

The compounds for use in the present invention are inositol phosphates, as defined in the first aspect of the invention, as well as analogs and derivatives thereof. The term "inositol phosphate" as used herein refers to a compound with an inositol ring and one, two, three, four, five, or six phosphate groups, or a combination thereof. Myo-inositol hexaphosphate (IP6) is an exemplary inositol phosphate of the present invention. In some aspects, the inositol phosphate is pure (e.g., over 99% of the inositol phosphate species are the same species, for example, IP6) or substantially pure (e.g., at least 90%, 91%, 92%, 93%, 94%, 95%, 96%, 97%, 98% or 99% of the inositol phosphate species are the same species, for example, IP6). In some aspects, the inositol phosphate is a mixture, e.g., comprising variable amounts of IP1, IP2, IP3, IP4, IP5, and IP6. In some aspects, the inositol phosphate is a racemic mixture.

The invention also contemplates inositol phosphate analogs. "Inositol phosphate analog" as used herein refers to a compound that has a ring with different number of carbons with respect to an inositol ring (i.e., 5 or 7 carbons), and/or has at least one sulfate or thiophosphate group. For example, a compound comprising a ring with 5, 6, or 7 carbons and at least one phosphate, sulfate, or thiophosphate group would be considered an inositol phosphate analog.

The term "inositol phosphate derivative" as used herein refers to an inositol phosphate or inositol phosphate analog which contains a heterologous moiety (i.e., a group that is not a phosphate, a sulfate, or a thiophosphate). For example, an inositol pentasulfate comprising a polyethylene glycol heterologous moiety, or a myo-inositol hexaphosphate comprising a polyglycerol heterologous moiety would be considered inositol phosphate derivatives.

The term "heterologous moiety" as used herein refers to a radical in the compound of formula I which is not a phosphate, a sulfate, or a thiophosphate, and confers a desirable property to such compound. For example, a heterologous moiety (e.g., a polyglycerol or a polyethyleneglycol) can increase the solubility of the compound. In some aspects, a heterologous moiety can confer multiple desirable properties (e.g., polyglycerol and polyethyleneglycol can both increase the solubility of a compound and reduce the clearance rate of the compound).

The terms "inositol phosphate of the invention" and "inositol phosphate of the present invention" as used herein is a generic term encompassing "inositol phosphate", "inositol phosphate analog", "inositol phosphate derivative" and combinations thereof. In some aspects, the term "inositol phosphate of the present invention" encompasses pharmaceutical compositions comprising an "inositol phosphate" an "inositol phosphate analog" an "inositol phosphate derivative" or a combination thereof, and pharmaceutically acceptable excipients and carriers. In some other aspects, the term "inositol phosphate of the present invention" encompasses combined preparations comprising an "inositol phosphate" an "inositol phosphate analog" an "inositol phosphate derivative" or a combination thereof, and at least a second active agent.

The terms "inositol phosphate of the invention" and "inositol phosphate of the present invention" as used herein is a generic term encompassing "inositol phosphate", "inositol phosphate analog", "inositol phosphate derivative" and combinations thereof. In some aspects, the term "inositol phosphate of the present invention" encompasses compositions comprising an "inositol phosphate" an "inositol phosphate analog" an "inositol phosphate derivative" or a combination thereof, and at least one second active agent.

Compounds of the present invention comprising a ring with 5, 6, or 7 carbons and at least one sulfate, or thiophosphate group but without a phosphate group would still be considered an "inositol phosphate analog" or an "inositol phosphate analog" in the context of the present invention. Thus, the term "inositol phosphate of the present invention" encompasses not only phosphate-containing compounds but also compounds without phosphate groups that comprise a ring with 5, 6, or 7 carbons and at least one sulfate, or thiophosphate group.

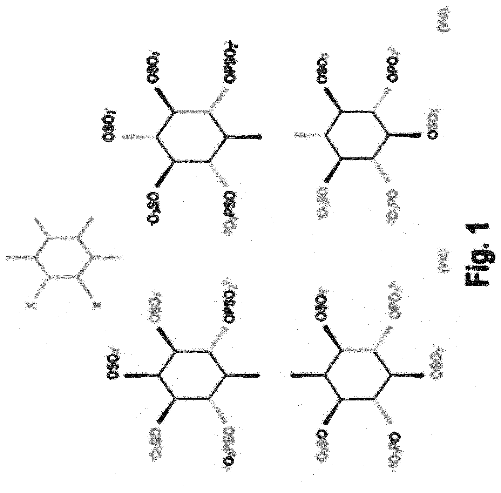

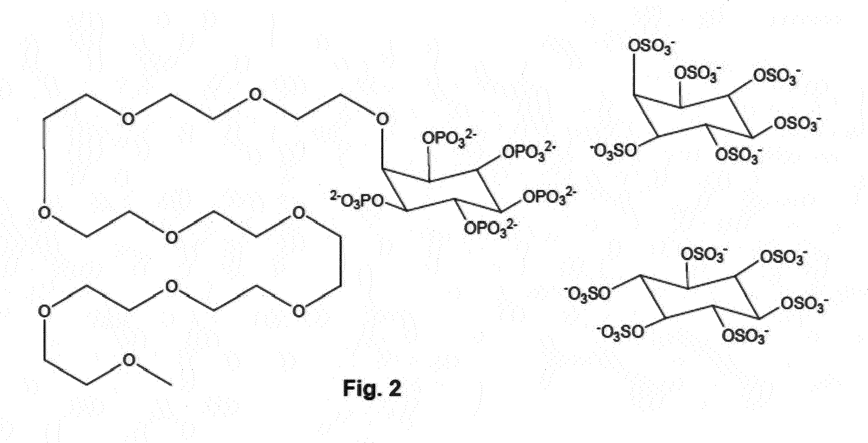

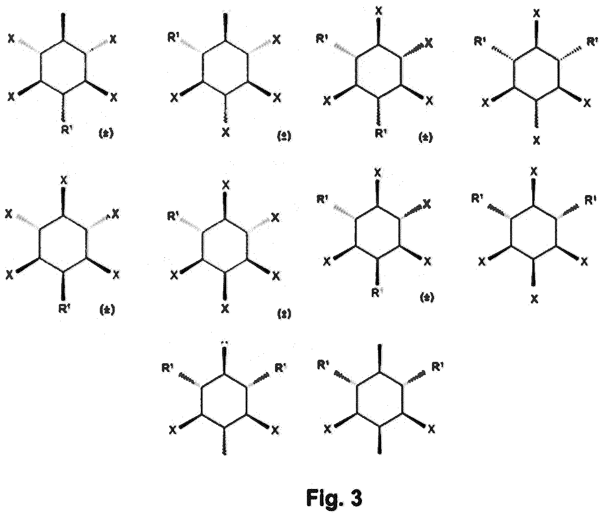

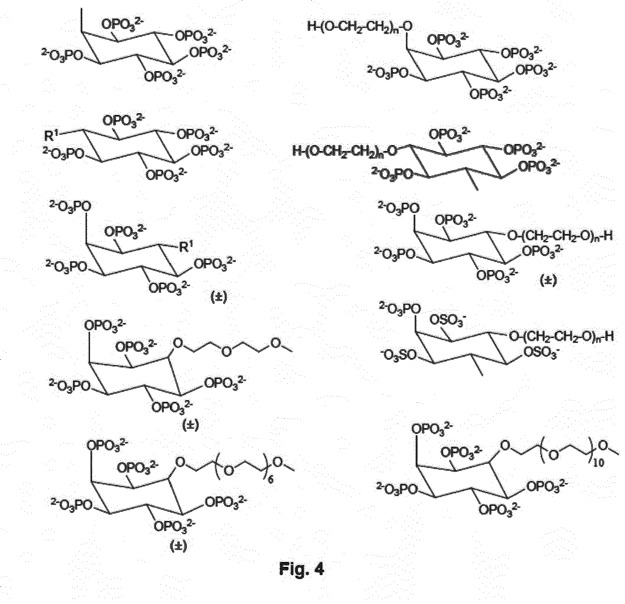

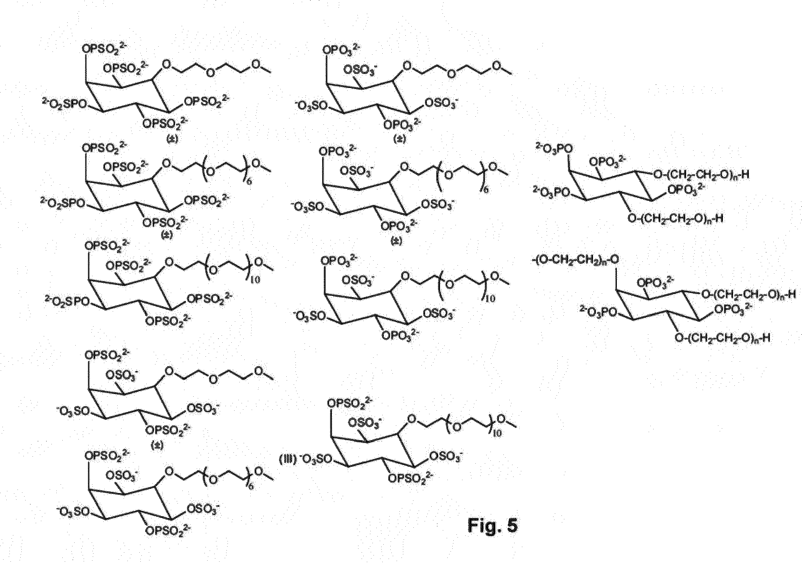

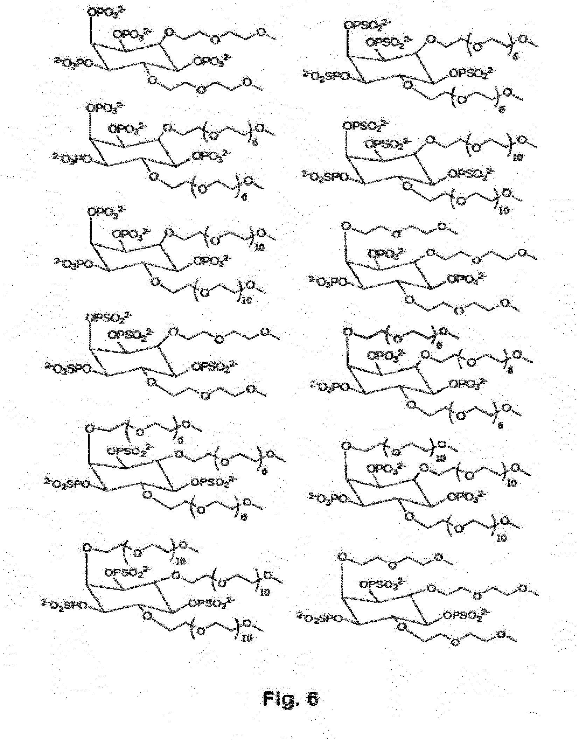

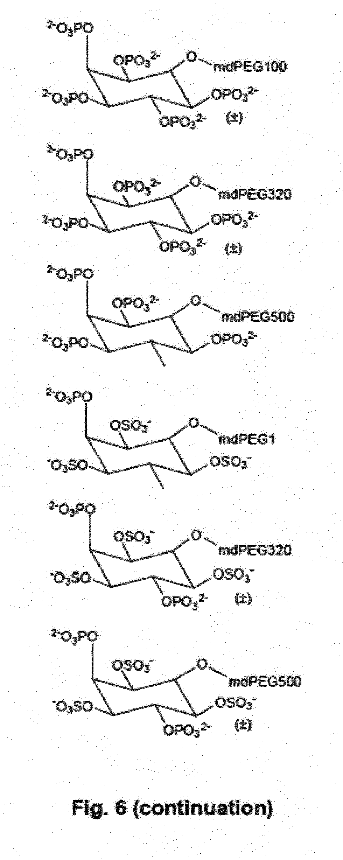

Representative inositol phosphates of the present invention are presented in **Figs. 1-6****.** **Fig. 3** presents numerous examples of inositol phosphates, all of them in the myo conformation. Besides myo-inositol, the other naturally occurring stereoisomers of inositol are scyllo-, muco-, 1D-chiro-, 1L-chiro-, neo-inositol, allo-, epi-, and cis-inositol. As their names denote, 1L- and 1D-chiro inositol are the only pair of inositol enantiomers, but they are enantiomers of each other, not of myo-inositol. It is to be understood that any exemplary inositol phosphate presented in the disclosure is not limited to the representative conformation displayed. Thus, for example, the examples presented in **Fig. 3** would also encompass the corresponding equivalents in scyllo-, muco-, 1D-chiro-, 1L-chiro-, neo-inositol, allo-, epi-, and cis-inositol conformations. In its most stable conformation, the myo-inositol isomer assumes the chair conformation, which moves the maximum number of hydroxyls to the equatorial position, where they are farthest apart from each other. In this conformation, the natural myo isomer has a structure in which five of the six hydroxyls (the first, third, fourth, fifth, and sixth) are equatorial, whereas the second hydroxyl group is axial.

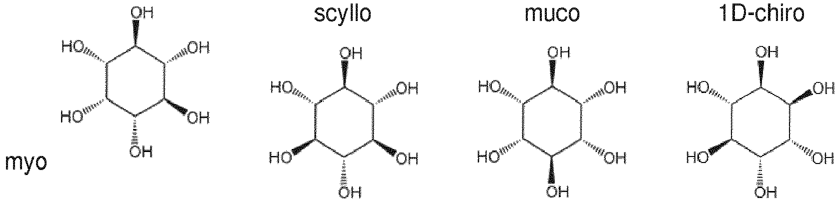

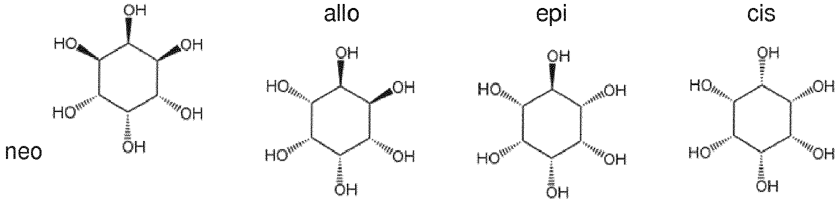

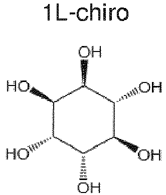

### Inositol phosphates, analogs and derivatives

In some aspects, at least one of R₁, R₃, R₅, R₇, R₉, and R₁₁ of the compound of general formula I independently represents H, -X, -OX, -NHX, -NX₂, -SX, -OSO₃HX, - OSO₃X₂ or a compound of formula II, formula III or formula IV, where each X independently represents H, C₁₋₃₀ alkyl, C₂₋₃₀ alkynyl or Cy₁, where C₁₋₃₀ alkyl, C₂₋₃₀ alkenyl and C₂₋₃₀ alkynyl are independently optionally substituted with one or more R₁₄ and where Cy₁ is optionally substituted by one or more R₁₅; Cy₁ represents a carbocyclic or heterocyclic three- to 10-membered ring, which can be saturated, partially unsaturated or aromatic, where said heterocycle has between one and four heteroatoms selected from amongst O, S and N, where said ring can be bound to the rest of the molecule via any available C atom and where Cy₁ is optionally fused to between one and four five- or six-membered rings, each saturated, partially unsaturated or aromatic, carbocyclic or heterocyclic, and where said fused heterocycle can contain one or two heteroatoms selected from amongst O, N and S; each R₁₃ independently represents H, C₁₋₃₀ alkyl, -NH₂, -NHC₁₋₃₀ alkyl or N(C₁₋₃₀ alkyl)₂, where each C₁₋₃₀ alkyl is independently optionally substituted with one or more halogen, -OH, -CN and -NO₂ groups; and each R₁₄ and R₁₅ independently represents -OH, C₁₋₃₀ alkoxy, C₁₋₃₀ alkyithionyl, C₁₋₃₀ acyloxy, phosphate, halogen, trihalo C₁₋₃₀ alkyl, nitrile azide.

In some ulterior aspects, each X independently represents H, C₁₋₃₀ alkyl or Cy₁, where C₁₋₃₀ alkyl is optionally substituted by one or more R₁₄ and where Cy₁ is optionally substituted by one or more R₁₅; and each R₁₄ and R₁₅ independently represents -OH, C₁₋₃₀ alkoxy, C₁₋₃₀ alkyithionyl, C₁₋₃₀ acyloxy, phosphate, halogen, trihaloC₁₋₃₀alkyl, nitrile or azide. In some aspects, each X represents H, C₁₋₃₀ alkyl or Cy₁. In some aspects, each X represents H.

In some additional aspects, at least one of radicals R₁, R₃, R₅, R₇, R₉ and R₁₁ independently represents a compound of formula II, formula III or formula IV, each R₁₃ independently represents H, C₁₋₃₀ alkyl, -NH₂, -NHC₁₋₃₀ alkyl or -N(C₁₋₃₀ alkyl)₂, where each C₁₋₃₀ alkyl is independently optionally substituted by one or more halogen, -OH, -CN and -NO₂ groups; and R₂, R₄, R₆, R₈, R₁₀, and R₁₂ independently represent H.

In a further aspect, R₁, R₃, R₅, R₇, R₉, and R₁₁ independently represent a compound of formula II, formula III, or formula IV, each R₁₃ independently represents H or C₁₋₃₀ alkyl, where each C₁₋₃₀ alkyl is independently optionally substituted by one or more halogen, -OH, -CN and -NO₂ groups; and R₂, R₄, R₆, R₈, R₁₀, and R₁₂ independently represent H.

In an additional aspect, at least one of R₁, R₃, R₅, R₇, R₉, and R₁₁ represent a compound of formula II, formula III, or formula IV, and each R₁₃ independently represents H or C₁₋₃₀ alkyl. In another aspect, at least one of R₁, R₃, R₅, R₇, R₉, and R₁₁ represent a compound of formula II, formula III or formula IV, and each R₁₃ represents H.

In a particular aspect, the compound is inositol hexaphosphate (IP6). In other aspects, the compound is inositol monophosphate (IP1), inositol diphosphate (IP2), inositol triphosphate (IP3), inositol tetraphosphate (IP4), or inositol pentaphosphate (IP5). In some aspects, the compound comprises a combination of IP1, IP2, IP3, IP4, IP5 and/or IP6. In some aspects, the IP6 can form other inositol phosphates (IP5, IP4, IP3, IP2, IP1) by dephosphorylation *in vivo.* Inositol is assumed to mean any isomeric form of the molecule, for example, myoinositol.

In some aspects, the compounds for use in the present invention are those of formula I wherein:
R₇ is OSO₃⁻, and R₉, R₅, R₃, R₁ and R₁₁ are independently selected from OPO₃²⁻, OPSO₂²⁻ or OSO₃⁻;
R₉, R₅ and R₁ are OPO₃²⁻ and R₇, R₃ and R₁₁ are OSO₃⁻;
R₉, R₅ and R₁ are OSO₃⁻ and R₇, R₃ and R₁₁ are OPO₃²⁻;
R₃, R₁ and R₁₁ are OSO₃⁻ and R₉, R₇ and R₅ are OPO₃²⁻;
R₃, R₁ and R₁₁ are OPO₃²⁻ and R₉, R₇ and R₅ are OSO₃⁻;
R₇ and R₁ are OPO₃²⁻ and R₉, R₅, R₃, and R₁₁ are OPO₃²⁻;
R₇ and R₁ are OSO₃⁻ and R₉, R₅, R₃, and R₁₁ are OPO₃²⁻;
R₇ and R₅ are OPO₃²⁻ and R₉, R₃, R₁, and R₁₁ are OSO₃⁻; or,
R₇ and R₅ are OSO₃⁻ and R₉, R₃, R₁, and R₁₁ are OPO₃²⁻.

The inositol phosphates of the present invention also encompass compounds that are produced as metabolites during physiological dephosphorylation (or desulfation or dethiosulfation in the case of compounds comprising sulfate or thiophosphate groups).

In some aspects, the compound administered in a dosage according to the methods disclosed herein is a prodrug that after undergoing hydrolysis or other intracellular or extracellular processing yields an inositol phosphate of the present invention.

The inositol phosphates of the present invention encompass also any combination of the inositol phosphate, inositol phosphate analogs, and derivatives thereof disclosed herein.

All compounds of formula I contain radicals with C-O-P or C-O-S bonds, which provide the compounds with an affinity for calcium-containing crystals and a sufficiently labile bond to be hydrolyzed *in vivo,* thereby preventing irreversible binding to calcium-containing crystals such as the hydroxyapatite (HAP) in bone, which would have a negative impact on bone remodeling, as is the case with bisphosphonates when administered long term as said compounds contain P-C-P bonds that cannot be hydrolyzed by the body. At the other extreme are phosphorylated compounds that do not contain said C-O-P bonds, such as pyrophosphates, the P-O-P bonds of which mean that they are too readily hydrolyzed in the intestine, thus meaning that only parenteral administration is feasible. The compounds of the present invention, with CO-P bonds, C-O-S bonds, and combinations thereof represent an adequate midpoint due to the efficacy thereof and the fact that the body presents mechanisms for eliminating said compounds, thus reducing the risk of side effects (e.g., compounds with P-C-P bonds can present half-lives of several months which *in vivo,* thereby affecting, e.g., bone remodeling).

The term "alkyl" or "alkyl group" in the context of the present invention refers to a saturated hydrocarbon moiety, which can be linear, branched, cyclic or cyclic with linear or branched side chains. The term alkyl includes partially unsaturated hydrocarbons such as propenyl. Examples are methyl, ethyl, n- or isobutyl, n- or cyclohexyl. The term alkyl can extend to alkyl groups linked or bridged by hetero atoms. Hetero atoms in the context of the present invention are nitrogen (N), sulfur (S) and oxygen (O).

An "amine function" or "amine group" is a function NR'R", with R' and R" selected independently from hydrogen and C₁-C₅ alkyl. In some aspects, R' and R" are selected from hydrogen and C₁-C₃ alkyl. A "hydroxy function" or "hydroxy group" is OH.

A "thiol function" or "thiol group" is SH. A "carboxylic acid function" or "carboxylic acid group" is COOH or its anion, COO⁻. A "carboxylic amide" is CONR'R", with R' and R" independently having the meanings indicated above. A "sulfonic acid" is SO₃H. A "sulfonic acid amide" is SO₂NR'R", with R' and R" independently having the meanings indicated above.

A "C₁-C₃ alkyl" in the context of the present invention refers to a saturated linear or branched hydrocarbon having 1, 2, or 3 carbon atoms, wherein one carbon-carbon bond can be unsaturated and one CH₂ moiety can be exchanged for oxygen (ether bridge). Non-limiting examples for a C₁-C₃ alkyl are methyl, ethyl, propyl, prop-2-enyl and prop-2-inyl.

A "C₁-C₅ alkyl" in the context of the present invention refers to a saturated linear or branched hydrocarbon having 1, 2, 3, 4 or 5 carbon atoms, wherein one or two carbon-carbon bond can be unsaturated and one CH₂ moiety can be exchanged for oxygen (ether bridge). Non-limiting examples for a C₁-C₅ alkyl include the examples given for C₁-C₃ alkyl above, and additionally n-butyl, 2-methylpropyl, tert-butyl, 3-methylbut-2-enyl, 2-methylbut-3-enyl, 3-methylbut-3-enyl, n-pentyl, 2-methylbutyl, 3-methylbutyl, 1,1-dimethylpropyl, 1,2-dimethylpropyl, 1,2-dimethylpropyl, but-3-enyl, but-3-inyl and pent-4-inyl. A "C₃-C₁₀ alkyl" in the context of the present invention refers to a saturated linear or branched hydrocarbon having 3, 4, 5, 6, 7, 8, 9 or 10 carbon atoms, wherein 1, 2 or 3 carbon-carbon bonds can be unsaturated and one CH₂ moiety can be exchanged for oxygen (ether bridge).

The term "C₁₋₃₀ alkyl" as a group or part of a group, refers to a linear or branched chain alkyl group containing between 1 and 30 carbon atoms including, amongst others, methyl, ethyl, propyl, isopropyl, butyl, isobutyl, sec-butyl, tert-butyl, pentyl, hexyl, decyl and dodecyl groups.

The term "C₂₋₃₀ alkenyl" refers to a linear or branched alkyl chain containing between 2 and 30 carbon atoms and also contains one or more double bonds. Examples include, amongst others, ethenyl, 1-propenyl, 2-propenyl, isopropenyl 1-butenyl, 2-butenyl, 3-butenyl and 1,3-butadienyl.

The term "C₂₋₃₀ alkynyl" refers to a linear or branched alkyl chain containing between 2 and 30 carbon atoms and also contains one or more triple bonds. Examples include, amongst others, ethynyl, 1-propynyl, 2-propynyl, 1-butynyl, 2-butynyl, 3-butynyl and 1,3-butadiynyl.

A "Cy₁ group" refers to a three- to 10-membered carbocyclic or heterocyclic ring that can be saturated, partially unsaturated or aromatic and which is bound to the rest of the molecule via any available C atom. When heterocyclic, Cy₁ contains between one and four heteroatoms selected from amongst N, O and S. Moreover, Cy₁ can optionally be fused with up to four five- or six-membered carbocyclic or heterocyclic rings, which can be saturated, partially unsaturated or aromatic. If the fused ring is a heterocycle, said ring contains one or two heteroatoms selected from amongst N, O and S. Examples of Cy₁ include, amongst others, phenyl, naphthyl, thienyl, furyl, pyrrolyl, thiazolyl, isothiazolyl, imidazolyl, pyrazolyl, 1,2,3-triazolyl, 1,2,4-triazolyl, tetrazolyl, 1,3,4-thiadiazolyl, 1,2,4-thiadiazolyl, pyridyl, pyrazinyl, pyrimidinyl, pyridazinyl, benzimidazolyl, benzofuranyl, isobenzofuranyl, indolyl, isoindolyl, benzothiophenyl, benzothiazolyl, cyclopropyl, cyclobutyl, cyclopentyl, cyclohexyl, cycloheptyl, azetidinyl and aziridinyl.

A "C₁₋₃₀ alkoxy group" as a group or part of a group refers to an -OC₁₋₃₀alkyl group, where the C₁₋₃₀alkyl part has the same meaning as above. Examples include methoxy, ethoxy, propoxy, isopropoxy, butoxy, isobutoxy, sec-butoxy and tert-butoxy.

A "C₁₋₃₀ alkylthionyl group" as a group or part of a group refers to an -SOC₁₋₃₀alkyl group, where the C₁₋₃₀alkyl part has the same meaning as above. Examples include methylthionyl, ethylthionyl, propyithionyl, isopropyithionyl, butylthionyl, isobutyithionyl, sec-butylthionyl and tert-butylthionyl.

A "C₁₋₃₀ acyloxy group" as a group or part of a group refers to a -COC₁₋₃₀alkyl group, where the C₁₋₃₀ alkyl part has the same meaning as above. Examples include acetyl, ethanoyl, propanoyl and 2,2-diisopropylpentanoyl.

A "halogen radical" or the halo abbreviation thereof refers to fluorine, chlorine, bromine and iodine.

A "trihalo C₁₋₃₀ alkyl group" refers to a group resulting from the substitution of three hydrogen atoms of a C₁₋₃₀ alkyl group by three halogen radicals as defined above. Examples include, amongst others, trifluoromethyl, tribromomethyl, trichloromethyl, triiodomethyl, trifluoroethyl, tribromoethyl, trichloroethyl, triiodoethyl, tribromopropyl, trichloropropyl and triiodopropyl.

An "-NHC₁₋₃₀ alkyl group" refers to a group resulting from the substitution of one hydrogen atom of an -NH₂ group by a C₁₋₃₀ alkyl group as defined above. Examples include, amongst others, methylamine, ethylamine, propylamine, butylamine and pentylamine.

A "-N(C₁₋₃₀ alkyl)₂ group" refers to a group resulting from the substitution of two hydrogen atoms of an -NH₂ group by a C₁₋₃₀ alkyl group as defined above. Examples include, amongst others, dimethylamine, diethylamine, diisopropylamine, dibutylamine and diisobutylamine.

The expression "optionally substituted by one or more" signifies the possibility that a group can be substituted by one or more (e.g., by 1, 2, 3 or 4) substituents. In some aspects, a group can be substituted by 1, 2 or 3 substituents and even by 1 or 2 substituents provided that the group has sufficient positions that can be substituted available. If present, the substituents can be the same or different and can be located at any available position.

In some aspects, the inositol phosphates of the present invention comprise the compounds disclosed in WO2017098033 and WO2017098047, and US US9358243. In some aspects, the inositol phosphates of the present invention used comprise the compounds disclosed in **Figs. 1-6****.**

In some aspects, the inositol phosphates, inositol phosphate analogs, and derivatives thereof, comprise compounds of formula (VIII), formula (IX), or formula (X): 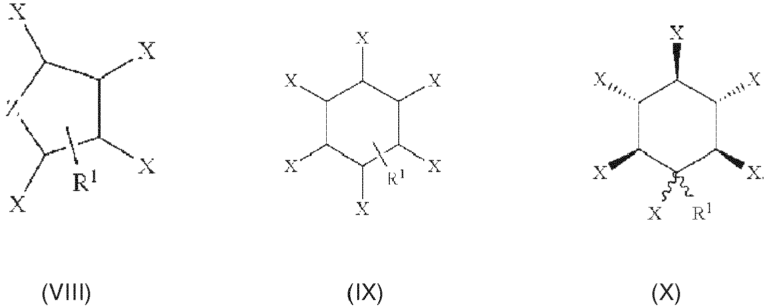 wherein each X independently is selected from OPO₃²⁻, OPSO₂²⁻, or OSO₃⁻; Z is an alkyl chain comprising 1 to 3 carbon and/or hetero atoms, optionally comprising a group X, wherein X is also selected from OPO₃²⁻, OPSO₂²⁻, or OSO₃⁻; and, R¹ is an optional heterologous moiety (See section 2.2. below). In some aspects, the molecule comprises more than one heterologous moiety, in which case the heterologous moieties can be the same or be different.

In some aspects, Z, as used in formula (VIII), is CH₂, CHX, CHR¹, CXR¹, CH₂-CH₂, CH₂-CHX, CHX-CHX, CHR¹-CHX, CXR¹-CHX, CHR¹-CH₂, CXR¹-CH₂, CHR¹-CHOH, CH₂-CH₂-CH₂, CH₂-O-CH₂, CHOH-CH₂-CH₂, CHOH-CHOH-CHR¹, CHOH-CHR¹-CHOH, CHX-CH₂-CH₂, CH₂-CHX-CH₂, CHX-CHX-CH₂, CHX-CH₂-CHX or CHX-CHR¹-CHX, wherein X independently is selected from OPO₃²⁻, OPSO₂²⁻, and OSO₃⁻.

In some aspects, Z, as used in formula (VIII), is (CHX)ₚCHX(CHX)_{q}; wherein p and q each independently from the other have a value from 0 to 2, with the proviso that (p+q) has a value of 0, 1 or 2; one or two or three X can be a heterologous moiety (e.g., PEG) and the remaining X are independently selected from OPO₃²⁻, OPSO₂²⁻, and OSO₃⁻. In some aspects, not all X of Z are OPO₃²⁻. In some aspects, not all X of Z are OSO₃⁻.

In some aspects, one, two, or three of the X in compounds of formula (VIII), formula (IX), or formula (X) can be heterologous moiety and the remaining X can independently be selected from OPO₃²⁻, OPSO₂²⁻, or OSO₃⁻.

Formula (VII) above describes a five-membered, six-membered, or seven-membered alkyl ring, and the optional heterologous moiety or moieties is/are attached to one of the carbon atoms forming the ring.

In some aspects, the inositol phosphates, inositol phosphate analogs, and derivatives thereof used, e.g., in the methods and compositions disclosed herein, comprise compounds of formula (XI) or formula (XII): 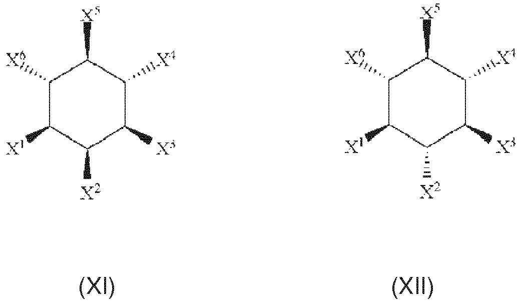 wherein:
X² is OSO₃⁻, and X¹, X³, X⁴, X⁵ and X⁶ are independently selected from OPO₃²⁻, OPSO₂²⁻ or OSO₃⁻;
X¹, X³ and X⁵ are OPO₃²⁻ and X², X⁴ and X⁶ are OSO₃⁻;
X¹, X³ and X⁵ are OSO₃⁻ and X², X⁴ and X⁶ are OPO₃²⁻ ;
X⁴, X⁵ and X⁶ are OSO₃⁻ and X¹, X² and X³ are OPO₃²⁻;
X⁴, X⁵ and X⁶ are OPO₃²⁻ and X¹, X² and X³ are OSO₃⁻;
X² and X⁵ are OPO₃²⁻ and X¹, X³, X⁴, and X⁶ are OPO₃²⁻;
X² and X⁵ are OSO₃⁻ and X¹, X³, X⁴, and X⁶ are OPO₃²⁻;
X² and X³ are OPO₃²⁻ and X¹, X⁴, X⁵, and X⁶ are OSO₃⁻; or,
X² and X³ are OSO₃⁻ and X¹, X⁴, X⁵, and X⁶ are OPO₃²⁻.

In some aspects, the inositol phosphates of the present invention or metabolites thereof can be detected and/or quantified using the methods disclosed in US9612250. See also, US8377909, US8778912, and US20070066574.

The compounds disclosed herein can be present in any form commonly used in pharmaceutical technology. Particular aspects include, but are not limited to, the sodium salt, magnesium salt, potassium salt, ammonium salt, free acid, or a mixture of the preceding forms. Other pharmaceutically acceptable salts are known to the skilled artisan and can be readily obtained. In a particular aspect, the compound for use as defined in the first aspect of the invention is a sodium salt, for example, inositol hexaphosphate hexasodium.

The present invention also contemplates sodium salts of inositol monophosphate, inositol diphosphate, inositol triphosphate, inositol tetraphosphate and inositol pentaphosphate in any of inositol isomeric forms, in particular, myo-inositol. A particular example of the compounds for use in the present invention is myo-inositol hexaphosphate hexasodium salt.

### Heterologous moiety

In some aspects, the present invention refers to a compound of general formula I, as defined above, wherein the heterologous moiety is selected from a radical of formula V, a radical of formula VI and a radical of formula VII: 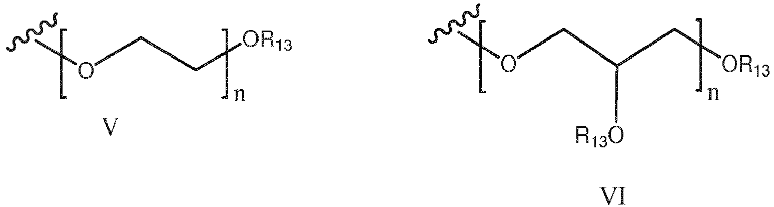 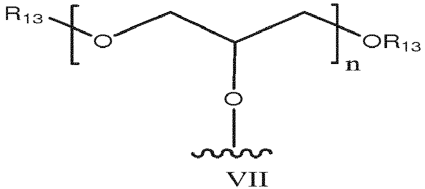 and wherein n is an integer in the range from 2 to 200, and R₁₃ is selected from H, methyl, or ethyl.

In some aspects, compounds for use in the present invention, for example, the inositol phosphate derivatives of the present invention, can comprise one or two radicals selected from the radicals of formulas V, VI and VII. These radicals are heterologous moieties conferring an advantageous property with respect to a corresponding molecule lacking such heterologous moiety or moieties. Examples of said advantageous properties that can be conferred by a heterologous moiety or a combination thereof to an inositol phosphate or inositol phosphate analogs include, but are not limited to (a) an increase in solubility, (b) a decrease in degradation or metabolization rate, (c) an increase in plasma half-life, (d) a decrease in liver metabolization rate (e) a decrease in clearance rate, (f) a decrease of toxicity, (g) a decrease of irritability and (h) reduced side effects among others. These advantageous properties can be evaluated or quantified using methods known in the art without undue experimentation.

In some aspects, the heterologous moiety is, for instance, a polyethylene glycol (PEG) or a polyglycerol (PG). Thus, in certain aspects, the compound for use in the invention is any of the compounds as defined in the aspects disclosed above comprising a heterologous moiety, that is, one of the radicals of formula I is selected from the radicals of formulas V, VI and VII. In some aspects the heterologous moiety comprises a polyethylene glycol (PEG). In certain aspects the heterologous moiety consists of polyethylene glycol, that is to say, at least one of R₁, R₃, R₅, R₇, R₉, and R₁₁ of the compound of formula I according to the first aspect of the invention is a radical of formula V. Alternatively, the heterologous moiety comprises a polyglycerol. In certain aspects the heterologous moiety consists on polyglycerol, that is to say, at least one of R₁, R₃, R₅, R₇, R₉, and R₁₁ of the compound of formula I according to the first aspect of the invention is selected from a radical of formula VI or VII. In other aspects the compound of formula I according to the first aspect of the invention contains one, two or three radicals selected from a radical of formula VI or VII, for example two PEGs (radical of formula V), Three PEGs, two polyglycerols (radical of formula VI), three PGs, or any combinations thereof, for example, one PEG and one PG, or two PEGs and one polyglycerol. In certain aspects all of the remaining radicals of formula I (i.e., those that are not a radical selected from V, VI and VII) are a radical selected from II, III and IV. In some aspects the compound of formula I according to the first aspect of the invention contains two radicals selected from a radical of formula VI or VII, for example two PEGs (radical of formula V) or two polyglycerols (radical of formula VI) or one PEG and one polyglycerol and the remaining radicals are all a radical of formula II. In some aspects R₃ and R₇ of the compound of formula I are selected from a radical of formula V, VI and VII. In some aspects, R₃ and R₇ of the compound of formula I are radicals of formula V, and R₁, R₅, R₉, and R₁₁ of the compound of formula I are radicals of formula II.

The radicals of formulas V, VI and VII have R₁₃ = H, methyl, or ethyl and n is an integer from 2 to 200. In some aspects R₁₃ = H. In particular aspects n is 2, 3, 4, 5, 6, 7, 8, 9, 10, 11, 12, 13, 14, 15, 16, 17,18,19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 189, 181, 182, 183, 184, 185, 186, 187, 188, 189, 190, 191, 192, 193, 194, 195, 196, 197, 198, 199, or 200.

In some aspects, n is between 2 and 10, between 10 and 20, between 20 and 30, between 30 and 40, between 40 and 50, between 50 and 60, between 60 and 70, between 70 and 80, between 80 and 90, between 90 and 100, between 100 and 110, between 110 and 120, between 120 and 130, between 130 and 140, between 140 and 150, between 150 and 160, between 160 and 170, between 170 and 180, between 180 and 190, or between 190 and 200.

In some specific aspects, n has a value from 2 to 200, from 2 to 20, from 10 to 30, or from 9 to 45.

In some aspects, the PEG is a branched PEG. Branched PEGs have three to ten PEG chains emanating from a central core group.

In certain aspects, the PEG moiety is a monodisperse polyethylene glycol. In the context of the present invention, a monodisperse polyethylene glycol (mdPEG) is a PEG that has a single, defined chain length and molecular weight. mdPEGs are typically generated by separation from the polymerization mixture by chromatography. In certain formulae, a monodisperse PEG moiety is assigned the abbreviation mdPEG. In some aspects, the PEG is a Star PEG. Star PEGs have 10 to 100 PEG chains emanating from a central core group.

In some aspects, the PEG is a Comb PEGs. Comb PEGs have multiple PEG chains normally grafted onto a polymer backbone.

In certain aspects, the PEG has a molar mass between 100 g/mol and 3000 g/mol, particularly between 100 g/mol and 2500 g/mol, more particularly of approx. 100 g/mol to 2000 g/mol. In certain aspects, the PEG has a molar mass between 200 g/mol and 3000 g/mol, particularly between 300 g/mol and 2500 g/mol, more particularly of approx. 400 g/mol to 2000 g/mol.

In some aspects, the PEG is PEG₁₀₀, PEG₂₀₀, PEG₃₀₀, PEG₄₀₀, PEG₅₀₀, PEG₆₀₀, PEG₇₀₀, PEG₃₀₀, PEG₉₀₀, PEG₁₀₀₀, PEG₁₁₀₀, PEG₁₂₀₀, PEG₁₃₀₀, PEG₁₄₀₀, PEG₁₅₀₀, PEG₁₆₀₀, PEG₁₇₀₀, PEG₁₈₀₀, PEG₁₉₀₀, PEG₂₀₀₀, PEG₂₁₀₀, PEG₂₂₀₀, PEG₂₃₀₀, PEG₂₄₀₀, PEG₂₅₀₀, PEG₁₆₀₀, PEG₁₇₀₀, PEG₁₈₀₀, PEG₁₉₀₀, PEG₂₀₀₀, PEG₂₁₀₀, PEG₂₂₀₀, PEG₂₃₀₀, PEG₂₄₀₀, PEG₂₅₀₀, PEG₂₆₀₀, PEG₂₇₀₀, PEG₂₈₀₀, PEG₂₉₀₀, ₒᵣ PEG₃₀₀₀. In one particular aspect, the PEG is PEG₄₀₀. In another particular aspect, the PEG is PEG₂₀₀₀.

In some aspects, the inositol phosphate of the invention is a mdPEG inositol phosphate derivative modified in positions 4 and 6. In some aspects, the inositol phosphate of the invention is inositol-1,2,3,5-tetraphosphate-4,6-bisPEG₂₀₀, inositol-1,2,3,5-tetraphosphate-4,6-bisPEG₃₀₀, inositol-1,2,3,5-tetraphosphate-4,6-bisPEG₄₀₀, inositol-1,2,3,5-tetraphosphate-4,6-bisPEG₅₀₀, inositol-1,2,3,5-tetraphosphate-4,6-bisPEG₆₀₀, inositol-1,2,3,5-tetraphosphate-4,6-bisPEG₇₀₀, inositol-1,2,3,5-tetraphosphate-4,6-bisPEG₈₀₀, inositol-1,2,3,5-tetraphosphate-4,6-bisPEG₉₀₀, or inositol-1,2,3,5-tetraphosphate-4,6-bisPEG₁₀₀₀. In some aspects, the inositol phosphate of the invention is inositol-1,2,3,5-tetraphosphate-4,6-bisPEG₁₀₀.

In other particular aspects R₃ and/or R₇ of the compound of formula I is a radical of formula V where R₁₃ is H and n is an integer from 9 to 45. In other particular aspects R₃ and R₇ of the compound of formula I is a radical of formula V where R₁₃ is H and n is an integer from 9 to 45 and R₁, R₅, R₉ and R₁₁ are all a radical of formula II. In other particular aspects the compound of formula I is a sodium salt with R₃ and R₇ = radical of formula V where R₁₃ is H and n is an integer from 9 to 45, and R₁, R₅, R₉, and R₁₁ are all a radical of formula II.

In some other aspects, the heterologous moiety is a polyglycerol (PG) described by the formula ((R₃-O-(CH₂-CHOH-CH₂O)ₙ-) with R₃ being hydrogen, methyl or ethyl, and n having a value from 3 to 200. In some aspects, n has a value from 3 to 20. In some aspects, n has a value from 10 to 30. In some alternatives of these aspects, n has a value from 9 to 45. In some aspects, the heterologous moiety is a branched polyglycerol described by the formula (R³-O-(CH₂-CHOR⁵-CH₂-O)ₙ-) with R⁵ being hydrogen or a linear glycerol chain described by the formula (R³-O-(CH₂-CHOH-CH₂-O)ₙ-) and R³ being hydrogen, methyl or ethyl. In some aspects, the heterologous moiety is a hyperbranched polyglycerol described by the formula (R³-O-(CH₂-CHOR⁵-CH₂-O)ₙ) with R⁵ being hydrogen or a glycerol chain described by the formula (R³-O-(CH₂-CHOR⁶-CH₂-O)ₙ-), with R⁶ being hydrogen or a glycerol chain described by the formula (R³-O-(CH₂-CHOR⁷-CH₂-O)ₙ-), with R⁷ being hydrogen or a linear glycerol chain described by the formula (R³-O-(CH₂-CHOH-CH₂-O)ₙ-) and R³ being hydrogen, methyl or ethyl. Hyperbranched glycerol and methods for its synthesis are known in the art (Oudshorn M, et al., Biomaterials 2006; 27:5471-5479, Wilms D, et al., Acc Chem Res 2010; 43:129-141) and references cited therein.

In certain aspects, the PG has a molar mass between 100 g/mol and 3000 g/mol, particularly between 100 g/mol and 2500 g/mol, more particularly of approx. 100 g/mol to 2000 g/mol. In certain aspects, the PG has a molar mass between 200 g/mol and 3000 g/mol, particularly between 300 g/mol and 2500 g/mol, more particularly of approx. 400 g/mol to 2000 g/mol.

In some aspects, the PG is PG₁₀₀, PG₂₀₀, PG₃₀₀, PG₄₀₀, PG₅₀₀, PG₆₀₀, PG₇₀₀, PG₈₀₀, PG₉₀₀, PG₁₀₀₀, PG₁₁₀₀, PG₁₂₀₀, PG₁₃₀₀, PG₁₄₀₀, PG₁₅₀₀, PG₁₆₀₀, PG₁₇₀₀, PG₁₈₀₀, PG₁₉₀₀, PG₂₀₀₀, PG₂₁₀₀, PG₂₂₀₀, PG₂₃₀₀, PG₂₄₀₀, PG₂₅₀₀, PG₁₆₀₀, PG₁₇₀₀, PG₁₈₀₀, PG₁₉₀₀, PG₂₀₀₀, PG₂₁₀₀, PG₂₂₀₀, PG₂₃₀₀, PG₂₄₀₀, PG₂₅₀₀, PG₂₆₀₀, PG₂₇₀₀, PG₂₈₀₀, PG₂₉₀₀, ₒᵣ PG₃₀₀₀. In one particular aspect, the PG is PG₄₀₀. In another particular aspect, the PG is PG₂₀₀₀.

In other particular aspects R₃ and/or R₇ of the compound of formula I is a radical of formula VI where R₁₃ is H and n is an integer from 9 to 45. In other particular aspects R₃ and R₇ of the compound of formula I is a radical of formula VI where R₁₃ is H and n is an integer from 9 to 45 and R₁, R₅, R₉, and R₁₁ are all a radical of formula II. In other particular aspects the compound of formula I is a sodium salt with R₃ and R₇ = radical of formula VI where R₁₃ is H and n is an integer from 9 to 45, and R₁, R₅, R₉, and R₁₁ are all a radical of formula II.

### Pharmaceutical compositions and combined preparations

In another aspect, the present invention also refers to pharmaceutical compositions comprising a compound, as defined in any of the aspects disclosed above. In some aspects, the pharmaceutical composition comprises a compound, as defined in any of the aspects disclosed above, together with one or more pharmaceutically acceptable excipients or carriers. In some aspects, these pharmaceutical compositions are for use in treating, inhibiting the progression, or preventing (a) cardiovascular calcification or (b) a disease, condition or symptom associated with cardiovascular calcification in a subject in need thereof, or (c) at risk of developing said cardiovascular calcification, associated disease, condition or symptom. In some additional aspects, the pharmaceutical compositions of the invention are for use in treating, inhibiting the progression, or preventing (a) coronary artery calcification, aortic artery calcification, or aortic valve calcification in subject in need thereof, or (b) a disease, condition or symptom associated to coronary artery calcification, aortic artery calcification, or aortic valve calcification, or (c) at risk of developing coronary artery calcification, aortic artery calcification, or aortic valve calcification or a disease, condition or symptom associated to any of them. In some further aspects, the pharmaceutical compositions of the inventions compose a combined preparation which comprises at least one second active agent.

Pharmaceutical compositions can comprise from approximately 1% to approximately 95% of the compound as defined in any of the aspects disclosed above. In some aspects, the pharmaceutical compositions of the present invention can comprise, for instance, from approximately 20% to approximately 90%, or from 20% to 80%, or from 20% to 70%, or from 20% to 60%, or from 20% to 50%, or from 30% to 90%, or from 40% to 90%, or from 50% to 90%, or from 60% to 90%, or from 30% to 70% of the compound as defined in any of the aspects disclosed above.

In some aspects, the concentration of inositol phosphate of the present invention (e.g., myo-inositol hexaphosphate or an analog or derivative thereof, or a combination thereof) in each dose of the pharmaceutical composition is between about 12.5 mM and about 135 mM. In some versions of this aspect, the concentration of inositol phosphate of the present invention (e.g., myo-inositol hexaphosphate or an analog or derivative thereof, or a combination thereof) in each dose of the pharmaceutical composition is about 25 mM, about 39 mM or about 114 mM.

Formulations of a pharmaceutical composition suitable for parenteral administration comprise a compound as defined in any of the aspects disclosed above mixed with a pharmaceutically acceptable carrier (e.g., as sterile water or sterile isotonic saline solution). Such formulations can be prepared, packaged, or sold in a form suitable for bolus administration or for continuous administration. Injectable formulations can be prepared, packaged, or sold in unit dosage form, such as in ampules or in multi-dose containers containing a preservative. Formulations for parenteral administration include, but are not limited to, suspensions, solutions, emulsions in oily or aqueous vehicles, pastes, and implantable sustained-release or biodegradable formulations. Such formulations can further comprise one or more additional ingredients including, but not limited to, suspending, stabilizing, or dispersing agents.

In some aspects, in a formulation for parenteral administration, the active agent (e.g., a compound as defined in any of the aspects disclosed above) is provided in dry (i.e., powder or granular) form for reconstitution with a suitable vehicle (e.g., sterile pyrogen-free water) prior to parenteral administration of the reconstituted composition.

The pharmaceutical compositions can be prepared, packaged, or sold in the form of a sterile injectable aqueous or oily suspension or solution. This suspension or solution can be formulated according to the known art, and may comprise, in addition to the active agent (e.g., compound as defined in any of the aspects disclosed above), additional ingredients such as the dispersing agents, wetting agents, or suspending agents described herein. Such sterile injectable formulations can be prepared using a non-toxic parenterally-acceptable diluent or solvent, such as water or 1,3-butanediol, for example. Other acceptable diluents and solvents include, but are not limited to, Ringer's solution, isotonic sodium chloride solution, and fixed oils such as synthetic mono- or di-glycerides.

Other parentally-administrable formulations which are useful include those which comprise the active agent (e.g., compound as defined in any of the aspects disclosed above) in microcrystalline form, in a liposomal preparation, or as a component of a biodegradable polymer system.

Compositions for sustained release or implantation can comprise pharmaceutically acceptable polymeric or hydrophobic materials such as an emulsion, an ion exchange resin, a sparingly soluble polymer, or a sparingly soluble salt.

Controlled- or sustained-release formulations of a pharmaceutical composition of the present invention can be made using conventional technology. In some cases, the dosage forms to be used can be provided as slow or controlled-release of one or more active agents therein using, for example, hydropropylmethyl cellulose, other polymer matrices, gels, permeable membranes, osmotic systems, multilayer coatings, microparticles, liposomes, or microspheres or a combination thereof to provide the desired release profile in varying proportions. Suitable controlled-release formulations known in the art, including those described herein, can be readily selected for use with the pharmaceutical compositions of the invention. Thus, single unit dosage forms suitable for parenteral or topical administration, such as injectable solutions, gels, creams, and ointments, which are adapted for controlled-release are encompassed by the present invention.

Most controlled-release pharmaceutical products have a common goal of improving therapy over that achieved by their non-controlled counterparts. Ideally, the use of an optimally designed controlled-release preparation in medical treatment is characterized by a minimum of active agent being employed to cure or control the condition in a minimum amount of time. Advantages of controlled-release formulations include extended activity of the active agent, reduced dosage frequency, and increased patient compliance. In addition, controlled-release formulations can be used to affect the time of onset of action or other characteristics, such as blood level of the active agent, and thus can affect the occurrence of side effects.

Most controlled-release formulations are designed to initially release an amount of active agent that promptly produces the desired therapeutic effect, and gradually and continually release of other amounts of active agent to maintain this level of therapeutic effect over an extended period of time. In order to maintain this constant level of active agent in the body, the active agent must be released from the dosage form at a rate that will replace the amount of active agent being metabolized and excreted from the body.

Controlled-release of an active agent can be stimulated by various inducers, for example pH, temperature, enzymes, water, or other physiological conditions or compounds. The term "controlled-release component" in the context of the present invention is defined herein as a compound or compounds, including, but not limited to, polymers, polymer matrices, gels, permeable membranes, liposomes, or microspheres or a combination thereof that facilitates the controlled-release of the active agent.

In certain aspects, the formulations of the present invention can be, but are not limited to, short-term, rapid-offset, as well as controlled, for example, sustained release, delayed release and pulsatile release formulations.

The term sustained release is used in its conventional sense to refer to active agent formulation (e.g., compound as defined in any of the aspects disclosed above)) that provides for gradual release of a therapeutic active agent over an extended period of time, and that can, although not necessarily, result in substantially constant blood levels of an active agent over an extended time period. The period of time can be as long as a month or more and should be a release which is longer that the same amount of agent administered in bolus form.

For sustained release, the compounds may be formulated with a suitable polymer or hydrophobic material which provides sustained release properties to the compounds. As such, the compounds for use the method of the present invention can be administered in the form of microparticles, for example, by injection or in the form of wafers or discs by implantation. In certain aspects, the compounds of the invention are administered to a patient, alone or in combination with another pharmaceutical agent, using a sustained release formulation.

The term delayed release is used herein in its conventional sense to refer to an active agent formulation that provides for an initial release of the active agent after some delay following active agent administration. The delay may be from about 10 minutes up to about 12 hours. The term pulsatile release is used herein in its conventional sense to refer to an active agent formulation that provides release of the active agent in such a way as to produce pulsed plasma profiles of the active agent after administration. The term immediate release is used in its conventional sense to refer to an active agent formulation that provides for release of the active agent immediately after administration.

In the context of the present invention, the term "non-bolus prolonged release form" refers to the administration of an active agent to a subject lasting for at least 1 minute, for a parenteral injection, for example an intravenous injection or for a subcutaneous injection, and for at least 30 minutes, for a parenteral infusion, for example an intravenous infusion administration.

Additional formulations and dosage forms of the compositions of the present invention include dosage forms as described in US6340475, US6488962, US6451808, US5972389, US5582837, and US5007790; US20030147952, 20030104062, 20030104053, 20030044466, 20030039688, and 20020051820; WO 2003035041, WO2003035040, WO2003035029, WO200335177, WO2003035039, WO2002096404, WO2002032416, WO2001097783, WO2001056544, WO2001032217, WO1998055107, WO1998011879, WO1997047285, WO1993018755, and WO1990011757.

Medicaments according to the invention are manufactured by methods known in the art, especially by conventional mixing, coating, granulating, dissolving or lyophilizing.

The present invention also provides a compound, a combination of compounds, or pharmaceutical formulation as defined in any of the above aspects of the invention, in the broadest definition given, or as specified in any of the aspects presented above, for use as a medicament.

A further aspect of the present invention relates to a combined preparation comprising (a) a compound and/or (b) a pharmaceutical composition according to present invention and (c) at least one second active agent. These second active agents are regularly administered to subjects with kidney failure. In some aspects, the combined preparation is administered to subjects undergoing dialysis. Some of these second active agents change the thermodynamics of the HAP crystallization process by modifying the concentration of the ions present in the structure of the calcium-containing crystal that is directly or indirectly responsible for the kidney failure-related disease.

Thus, in some aspects, the second active agent is selected from the group consisting of a vitamin, calcimimetic, bisphosphonate, phosphate binder, thiosulfate, pyrophosphate, citrate, diuretic, antihypertensive, anticholesteraemic agent, phosphodiesterase inhibitor, and combinations thereof.

In some aspects, the vitamin in the combined preparation is vitamin B, vitamin D, vitamin K, or a combination thereof. In some aspects, the calcimimetic in the combined preparation is cinacalcet ((R)-N-[1-(1-naphthyl)ethyl]-3-[3-(trifluoromethyl)phenyl]propan-1-amine, KAI-4169 (etelcalcetide), NPS R-467 (((R)-N-(3-phenylpropyl)-1-(3-methoxyphenyl)ethylamine)), NPS R-568 ((R)-2-chloro-N-(1-(3-methoxyphenyl)ethyl)benzenepropanamine), or a combination thereof. In some aspects, the bisphosphonate in the combined preparation may contain nitrogen or be nitrogen-free. In some additional aspects, the bisphosphonate is alendronate, clodronate, etidronate, ibandronate, monidronate, neridronate, olpadronate, pamidronate, parnidronate, risedronate, tiludronate, zoledronate, or a combination thereof.

In some aspects, the phosphate binder in the combined preparation is an aluminum salt, calcium salt, iron salt, lanthanum salt, magnesium salt, sevelamer salt, or a combination thereof. In some further aspects, the phosphodiesterase inhibitor in the combined preparation is cilostazol, pentoxifylline, or a combination thereof.

In some aspects, the diuretic in the combined preparation is a thiazide, thiazide-like (e.g., indapamide, chlortalidone, metolazone), loop diuretic (e.g., bumetanide, etacrynic acid, furosemide, torsemide), carbonic anhydrase inhibitor, osmotic diuretic, or potassium-sparing diuretic. In some further aspects, the thiazide is chlorothiazide, epithiazide, bendroflumethiazide, hydrochlorothiazide, or a combination thereof.

In some aspects, the antihypertensive in the combined preparation is a diuretic, an adrenergic blocker (e.g., beta-blocker, alpha-blocker, a combination thereof mixed), a calcium channel blocker (e.g., dihydropyridine), a renin inhibitor, an angiotensin-converting enzyme inhibitor, an angiotensin II receptor antagonist, an aldosterone antagonist, a vasodilator, an alpha-2 agonist or a blood pressure vaccine. In some further aspects, the anticholesteraemic agent in the combined preparation is a statin, a fibrate, niacin, a bile acid sequestrant, ezetimibe, lomitapide, a phytosterol, orlistat, or combination thereof.

In some aspects, the (a) a compound and/or (b) a pharmaceutical composition according to present invention and (c) the at least one second active agent of the combined preparation are administered separately, simultaneously or sequentially to a subject in need thereof. In some further aspects, the (a) a compound and/or (b) a pharmaceutical composition according to present invention and (c) the at least one second active agent of the combined preparation are mixed before their administration to the subject in need thereof.

### Methods and routes of administration

In some aspects, the administration of an effective amount of compound, pharmaceutical composition or combined preparation as defined in any of the aspects above is provided. Said compound, pharmaceutical composition or combined preparation can be administered parenterally such as, for example, intravenously, intraperitoneally, intramuscularly, intra-arterially, intradermal, intrathecal, epidural or spinal or subcutaneously. The parenteral administration may be by bolus injection or by intravenous infusion.

In a particular aspect of the present invention, myo-inositol hexaphosphate (or a formulation comprising myo-inositol hexaphosphate such as SNF472) is administered via intravenous infusion. In another particular aspect of the present invention, myo-inositol hexaphosphate is administered subcutaneously. In another aspect a derivative of inositol or myo-inositol hexaphosphate derivative, for example, a compound of formula I with R₃ and R₇ = radical of formula V where R₁₃ is H and n is an integer from 2 to 200, and R₁, R₅, R₉, and R₁₁ are all a radical of formula II (or a sodium salt thereof) is administered via intravenous infusion. In another aspect, a derivative of inositol or myo-inositol hexaphosphate derivative, for example, a compound of formula I with R₃ and R₇ = radical of formula V where R₁₃ is H and n is an integer from 2 to 200, and R₁, R₅, R₉, and R₁₁ are all a radical of formula II (or a sodium salt thereof) is administered subcutaneously.

In some aspects, the dose of inositol phosphate of the present invention comprises from about 200 mg to about 700 mg of an inositol phosphate, an inositol phosphate analog, an inositol phosphate derivative, or combination thereof per administration. In some aspects, when the disease or condition treated or prevented is related to coronary artery calcification, aortic artery calcification, or aortic valve calcification in a subject with kidney failure (e.g., stage 5 CKD patients), the dose is about 300 mg to 600 mg of the inositol phosphate of the invention per administration.

In some aspects, the dose of inositol phosphate of the present invention (e.g., myo-inositol hexaphosphate) is between about 200 mg and about 300 mg, between about 300 mg and about 400 mg, between about 400 mg and about 500 mg, between about 500 mg and about 600 mg, or between about 600 mg and about 700 mg per administration.

In some aspects, the dose of inositol phosphate of the present invention (e.g., myo-inositol hexaphosphate) is between about 200 mg and about 250 mg, between about 250 mg and about 300 mg, between about 300 mg and about 350 mg, between about 350 mg and about 400 mg, between about 400 mg and about 450 mg, between about 450 mg and about 500 mg, between about 500 mg and about 550 mg, between about 550 mg and about 600 mg, between about 650 mg and about 700 mg per administration.

In some aspects, the dose of inositol phosphate of the present invention (e.g., myo-inositol hexaphosphate) is between about 200 mg and about 400 mg, between about 300 mg and about 500 mg, between about 400 mg and about 600 mg, between about 500 mg and about 700 mg, between about 200 mg and about 500 mg, between about 300 mg and about 600 mg, or between 400 mg and about 700 mg per administration.

In some aspects, the dose of inositol phosphate of the present invention (e.g., myo-inositol hexaphosphate) is about 200 mg, about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 440 mg, about 450 mg, about 460 mg, about 470 mg, about 480 mg, about 490 mg, about 500 mg, about 510 mg, about 520 mg, about 530 mg, about 540 mg, about 550 mg, about 560 mg, about 570 mg, about 580 mg, about 590 mg, about 600 mg, about 610 mg, about 620 mg, about 630 mg, about 640 mg, about 650 mg, about 660 mg, about 670 mg, about 680 mg, about 690 mg, or about 700 mg per administration.

In some aspects, the dose of inositol phosphate of the present invention (e.g., myo-inositol hexaphosphate) is between about 210 mg and about 700 mg, between about 220 mg and about 700 mg, between about 230 mg and about 700 mg, between about 240 mg and about 700 mg, between about 250 mg and about 700 mg, between about 260 mg and about 700 mg, between about 270 mg and about 700 mg, between about 280 mg and about 700 mg, between about 290 mg and about 700 mg, between about 300 mg and about 700 mg, between about 310 mg and about 700 mg, between about 320 mg and about 700 mg, between about 330 mg and about 700 mg, between about 340 mg and about 700 mg, between about 350 mg and about 700 mg, between about 360 mg and about 700 mg, between about 370 mg and about 700 mg, between about 380 mg and about 700 mg, between about 390 mg and about 700 mg, between about 400 mg and about 700 mg, between about 410 mg and about 700 mg, between about 420 mg and about 700 mg, between about 430 mg and about 700 mg, between about 440 mg and about 700 mg, between about 450 mg and about 700 mg, between about 460 mg and about 700 mg, between about 470 mg and about 700 mg, between about 480 mg and about 700 mg, between about 490 mg and about 700 mg, between about 500 mg and about 700 mg, between about 510 mg and about 700 mg, between about 520 mg and about 700 mg, between about 530 mg and about 700 mg, between about 540 mg and about 700 mg, between about 550 mg and about 700 mg, between about 560 mg and about 700 mg, between about 570 mg and about 700 mg, between about 580 mg and about 700 mg, between about 590 mg and about 700 mg, between about 600 mg and about 700 mg, between about 610 mg and about 700 mg, between about 620 mg and about 700 mg, between about 630 mg and about 700 mg, between about 640 mg and about 700 mg, between about 650 mg and about 700 mg, between about 660 mg and about 700 mg, between about 670 mg and about 700 mg, between about 680 mg and about 700 mg, or between about 690 mg and about 700 mg per administration.

In some aspects, the dose of inositol phosphate of the present invention (e.g., myo-inositol hexaphosphate) is between about 200 mg and about 210 mg, between about 200 mg and about 220 mg, between about 200 mg and about 230 mg, between about 200 mg and about 240 mg, between about 200 mg and about 250 mg, between about 200 mg and about 260 mg, between about 200 mg and about 270 mg, between about 200 mg and about 280 mg, between about 200 mg and about 290 mg, between about 200 mg and about 300 mg, between about 200 mg and about 310 mg, between about 200 mg and about 320 mg, between about 200 mg and about 330 mg, between about 200 mg and about 340 mg, between about 200 mg and about 350 mg, between about 200 mg and about 360 mg, between about 200 mg and about 370 mg, between about 200 mg and about 380 mg, between about 200 mg and about 390 mg, between about 200 mg and about 400 mg, between about 200 mg and about 410 mg, between about 200 mg and about 420 mg, between about 200 mg and about 430 mg, between about 200 mg and about 440 mg, between about 200 mg and about 450 mg, between about 200 mg and about 460 mg, between about 200 mg and about 470 mg, between about 200 mg and about 480 mg, between about 200 mg and about 490 mg, between about 200 mg and about 500 mg, between about 200 mg and about 510 mg, between about 200 mg and about 520 mg, between about 200 mg and about 530 mg, between about 200 mg and about 540 mg, between about 200 mg and about 550 mg, between about 200 mg and about 560 mg, between about 200 mg and about 570 mg, between about 200 mg and about 580 mg, between about 200 mg and about 590 mg, between about 200 mg and about 600 mg, between about 200 mg and about 610 mg, between about 200 mg and about 620 mg, between about 200 mg and about 630 mg, between about 200 mg and about 640 mg, between about 200 mg and about 650 mg, between about 200 mg and about 660 mg, between about 200 mg and about 670 mg, between about 200 mg and about 680 mg, or between about 200 mg and about 690 mg per administration.

In some aspects, the dose of inositol phosphate of the present invention (e.g., myo-inositol hexaphosphate) is administered once daily (i.e., as a single daily dose). In some aspects, the daily dose can be subdivided into smaller doses and administered separately. Accordingly, in some aspect, the total daily dose can be subdivided into 2, 3, 4 or more sub-doses (i.e., multiple daily doses).

In some aspects, the dose of inositol phosphate of the present invention (e.g., myo-inositol hexaphosphate) is administered at least once a week. In some aspects, the dose of inositol phosphate of the present invention (e.g., myo-inositol hexaphosphate) is administered at least 2, at least 3, at least 4, at least 5, at least 6 or at least 7 times per week.

In some aspects, the dose of inositol phosphate of the present invention (e.g., myo-inositol hexaphosphate) is administered for at least one week. In some aspects, the dose of inositol phosphate of the present invention is administered for about 2 weeks, about 3 weeks, about 4 weeks, about 5 weeks, about 6 weeks, about 7 weeks, about 8 weeks, about 9 weeks, about 10 weeks, about 11 weeks, about 12 weeks, about 13 weeks, about 14 weeks, about 15 weeks, about 16 weeks, about 17 weeks, about 18 weeks, about 19 weeks, about 20 weeks, about 21 weeks, about 22 weeks, about 23 weeks, about 24 weeks, about 25 weeks, about 26 weeks, about 27 weeks, about 28 weeks, about 29 weeks, about 30 weeks, about 31 weeks, about 32 weeks, about 33 weeks, about 34 weeks, about 35 weeks, about 36 weeks, about 37 weeks, about 38 weeks, about 39 weeks, about 40 weeks, about 41 weeks, about 42 weeks, about 43 weeks, about 44 weeks, about 45 weeks, about 46 weeks, about 47 weeks, about 48 weeks, about 49 weeks, about 50 weeks, about 51 weeks, or about 52 weeks.

In some aspects, the dose of inositol phosphate of the present invention (e.g., myo-inositol hexaphosphate) is administered for at least 1 week, at least 2 weeks, at least 3 weeks, at least 4 weeks, at least 5 weeks, at least 6 weeks, at least 7 weeks, at least 8 weeks, at least 9 weeks, at least 10 weeks, at least 11 weeks, at least 12 weeks, at least 13 weeks, at least 14 weeks, at least 15 weeks, at least 16 weeks, at least 17 weeks, at least 18 weeks, at least 19 weeks, at least 20 weeks, at least 21 weeks, at least 22 weeks, at least 23 weeks, at least 24 weeks, at least 25 weeks, at least 26 weeks, at least 27 weeks, at least 28 weeks, at least 29 weeks, at least 30 weeks, at least 31 weeks, at least 32 weeks, at least 33 weeks, at least 34 weeks, at least 35 weeks, at least 36 weeks, at least 37 weeks, at least 38 weeks, at least 39 weeks, at least 40 weeks, at least 41 weeks, at least 42 weeks, at least 43 weeks, at least 44 weeks, at least 45 weeks, at least 46 weeks, at least 47 weeks, at least 48 weeks, at least 49 weeks, at least 50 weeks, at least 51 weeks, or at least 52 weeks.

In some aspects, the dose of inositol phosphate of the present invention (e.g., myo-inositol hexaphosphate) is administered for 1 week to 4 weeks, for 1 week to 8 weeks, for 1 week to 12 weeks, for 1 week to 16 weeks, for 1 week to 20 weeks, for 1 week to 24 weeks, for 1 week to 28 weeks, for 1 week to 32 weeks, for 4 weeks to 8 weeks, for 4 weeks to 12 weeks, for 4 weeks to 16 weeks, for 4 weeks to 20 weeks, for 4 weeks to 24 weeks, for 4 weeks to 28 weeks, for 4 weeks to 32 weeks, for 8 weeks to 12 weeks, for 8 weeks to 16 weeks, for 8 weeks to 20 weeks, for 8 weeks to 24 weeks, for 8 weeks to 28 weeks, for 8 weeks to 32 weeks, for 12 weeks to 16 weeks, for 12 weeks to 20 weeks, for 12 weeks to 24 weeks, for 12 weeks to 28 weeks, for 12 weeks to 32 weeks, for 16 weeks to 20 weeks, for 16 weeks to 24 weeks, for 16 weeks to 28 weeks, for 16 weeks to 32 weeks, for 20 weeks to 24 weeks, for 20 weeks to 28 weeks, for 20 weeks to 32 weeks, for 24 weeks to 28 weeks, for 24 weeks to 32 weeks, for 28 weeks to 32 weeks, for 32 weeks to 36 weeks, for 36 weeks to 40 weeks, for 40 weeks to 44 weeks, for 44 weeks to 48 weeks, or for 48 weeks to 52 weeks.

In a particular aspect, the dose of inositol phosphate of the present invention (e.g., a 300 mg or 600 mg dose of myo-inositol hexaphosphate) is administered 3 times per week. In a particular aspect, the dose of inositol phosphate of the present invention (e.g., a 300 mg or 600 mg dose of myo-inositol hexaphosphate) is administered 3 times per week for at least 12 weeks, for at least 24 weeks, for at least 42 weeks, or for at least 52 weeks.

Alternatively, the compound, pharmaceutical composition or combined preparation can be administered as a component of a hemodialysis, hemofiltration, or peritoneal dialysis solution or system.

In the particular case of patients treated with dialysis, a very appropriate method of administration consists of an administration (e.g., a non-bolus type administration) of an inositol phosphate of the present invention via the dialysis apparatus (before or after the filter) instead of directly injecting the inositol phosphate of the present invention into the patient intravenously. Thus, blood can be treated with the inositol phosphate of the present invention (e.g., myo-inositol hexaphosphate) as it leaves the patient and circulates through the dialysis circuit and, when the blood containing the inositol phosphate of the present invention returns to the body.

Thus, in some aspects, the compound, pharmaceutical composition or combined preparation, as defined in any of the aspects disclosed above, is administered to a patient during hemodialysis. In some aspects, the compound, pharmaceutical composition or combined preparation, as defined in any of the aspects disclosed above, is administered to the blood extracted from the patient during hemodialysis, preferably before it is filtered (i.e., the therapeutic agent is administered to the patient's unfiltered blood in the dialysis circuit). In some aspects, the compound is inositol hexaphosphate, in particular myo-inositol hexaphosphate sodium salt or a derivative thereof, namely, a compound of formula I with R₃ and R₇ = radical of formula V where R₁₃ is H and n is an integer from 2 to 200, and R₁, R₅, R₉ and R₁₁ are all a radical of formula II (or a sodium salt thereof).

In the case of dialysis patients, administration of an inositol phosphate of the present invention (e.g., myo-inositol hexaphosphate) via the dialysis apparatus allows the blood to equilibrate with the dialysis fluid prior to returning to the body; thus, although inositol phosphate of the present invention (e.g., myo-inositol hexaphosphate) can sequester ionic calcium, this fact is compensated when the blood passes through the dialysis filter thereby eliminating said side effect and significantly improving the safety profile. Additionally, administering the inositol phosphate of the present invention (e.g., myo-inositol hexaphosphate) concomitant to hemodialysis, in particular when administered to the unfiltered blood extracted from the patient during hemodialysis, allows for reducing the dose of the compound with consequent advantages in terms of reduced toxicity and minimizing adverse side effects.

In some aspects, the compound, pharmaceutical composition or combined preparation as defined in any of the aspects disclosed above is administered to a patient that is being treated with hemodialysis before the dialysis treatment or after a dialysis treatment.

### Indications

The compounds, pharmaceutical compositions, combined preparations, methods and routes of administration, as defined in any of the aspects disclosed above, can be used for treating, inhibiting the progression, or preventing (a) cardiovascular calcification or (b) a disease, condition or symptom associated with cardiovascular calcification in a subject in need thereof, or (c) at risk of developing said cardiovascular calcification, associated disease, condition or symptom. In some aspects, the subject is with kidney failure.

In some additional aspects, the compounds, pharmaceutical compositions, combined preparations, methods and routes of administration, as defined in any of the aspects disclosed above, can be used for treating, inhibiting the progression, or preventing (a) coronary artery calcification, aortic artery calcification, or aortic valve calcification, or (b) a disease, condition or symptom associated to coronary artery calcification, aortic artery calcification, or aortic valve calcification in subject in need thereof, or (c) at risk of developing coronary artery calcification, aortic artery calcification, or aortic valve calcification or a disease, condition or symptom associated to any of them. In some further aspects, the pharmaceutical compositions of the inventions compose a combined preparation which comprises at least one second active agent. In some aspects, the subject is with kidney failure.

In some aspects, the kidney disease in the subject can be acute, chronic or both. In some aspects, the subject is undergoing dialysis (e.g., peritoneal, hemodialysis). In a further form of this aspect, the subject is undergoing hemodialysis. In some other aspects, the subject is not dialyzed (e.g., a subject with CKD in stages 1 to 4).

The following embodiments further illustrate the scope of the invention:
Embodiment 1. A compound of general formula I, or a pharmaceutically acceptable salt thereof: 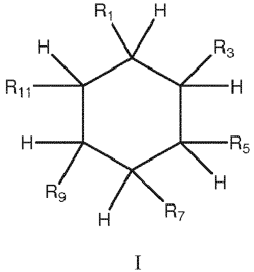 wherein:
   (i) R₁, R₃, R₅, R₇, R₉, and R₁₁ are independently selected from OH, a radical of formula II, III, IV and a heterologous moiety: 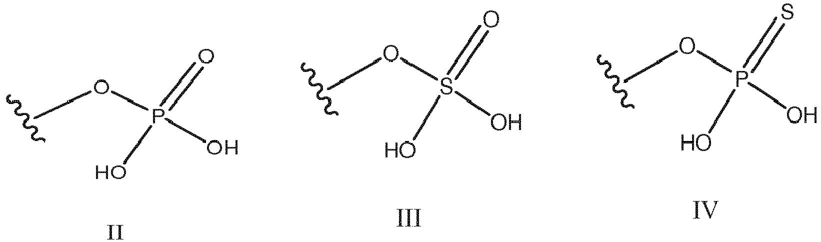
   (ii) at least one of R₁, R₃, R₅, R₇, R₉, and R₁₁ is selected from a radical of formula II, III and IV; and
   (iii) zero, one, two or three of R₁, R₃, R₅, R₇, R₉, and R₁₁ is a heterologous moiety;
   for use in the treatment, inhibition of progression, or prevention of cardiovascular calcification, or a disease, condition or symptom associated with cardiovascular calcification in a subject in need thereof, wherein (a) the compound is in a form suitable for parenteral, topical, or enteral administration, and (b) the compound is administered to the subject in a non-bolus prolonged release form in an effective dosage of about 200 mg to about 700 mg per administration.
Embodiment 2. The compound according to embodiment 1, wherein the heterologous moiety is selected from a radical of formula V, a radical of formula VI and a radical of formula VII: 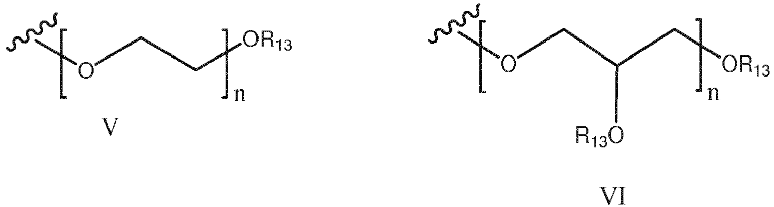 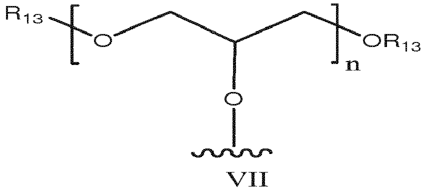 wherein n is an integer in the range from 2 to 200, and R₁₃ is selected from H, methyl, and ethyl.
Embodiment 3. The compound according to any one of embodiments 1-2, wherein the cardiovascular calcification is coronary artery calcification, aortic artery calcification, or aortic valve calcification.
Embodiment 4. The compound according to any one of embodiments 1-3, wherein the compound of formula I is inositol hexaphosphate.
Embodiment 5. The compound according to embodiment 4, wherein the inositol hexaphosphate is myo-inositol hexaphosphate.
Embodiment 6. The compound according to any one of embodiments 4-5, wherein the inositol hexaphosphate is in its hexasodium salt form.
Embodiment 7. The compound according to any one of embodiments 1-3, wherein one or two of R₁, R₃, R₅, R₇, R₉, and R₁₁ is selected from a radical of formula V, VI and VII.
Embodiment 8. The compound according to embodiment 7, wherein R₁, R₅, R₉ and R₁₁, are a radical of formula II and R₃ and R₇ are a radical of formula V.
Embodiment 9. The compound according to embodiment 8, wherein the radical of formula V has n in the range from 2 to 200 and R₁₃ is H.
Embodiment 10. A pharmaceutical composition for use in the treatment, inhibition of progression, or prevention of cardiovascular calcification in a subject in need thereof comprising the compound according to any one of embodiments 1-9 and pharmaceutically acceptable excipients and carriers.
Embodiment 11. The compound according to any one of embodiments 1-9 or the pharmaceutical composition according to embodiment 10, wherein the subject is with kidney failure.
Embodiment 12. The compound according to any one of embodiments 1-9 or the pharmaceutical composition according to embodiment 10, wherein the subject is on dialysis.
Embodiment 13. The compound or pharmaceutical composition according to any one of embodiments 1-12, wherein the parenteral administration is intravenous, subcutaneous, intramuscular or by intravenous infusion.
Embodiment 14. The compound or pharmaceutical composition according to embodiment 13, wherein the intravenous infusion is administered using a dialysis apparatus.
Embodiment 15. The compound or pharmaceutical composition according to any one of embodiments 1-14, wherein the subject is human.
Embodiment 16. The compound or pharmaceutical composition according to any one of embodiments 1-15, wherein the compound is administered to the subject in a dosage of about 250 mg to about 650 mg per administration to the subject.
Embodiment 17. The compound or pharmaceutical composition according to any one of embodiments 1-17, wherein compound is administered to the subject in a dosage about 300 mg to about 600 mg per administration to the subject.
Embodiment 18. The compound or pharmaceutical composition according to any one of embodiments 1-17, wherein compound is administered to the subject in a dosage of about 350 mg to about 550 mg per administration to the subject.
Embodiment 19. The compound or pharmaceutical composition according to any one of embodiments 1-18, wherein the dosage is administered in single daily doses.
Embodiment 20. The compound or pharmaceutical composition according to any one of embodiments 1-18, wherein the dosage is administered as multiple daily doses.
Embodiment 21. The compound or pharmaceutical composition according to any one of embodiments 1-20, wherein the dosage is administered at least once a week.
Embodiment 22. The compound or pharmaceutical composition according to any one of embodiments 1-21, wherein the dosage is administered 2, 3, 4, 5, 6 or 7 times per week.
Embodiment 23. The compound or pharmaceutical composition according to any one of embodiments 1-22, wherein the dosage is administered for at least one week.
Embodiment 24. The compound or pharmaceutical composition according to any one of embodiments 1-23, wherein the dosage is administered for about 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, or 52 weeks.
Embodiment 25. The compound or pharmaceutical composition according to any one of embodiments 1-24, wherein the dosage is administered for at least 12 weeks.
Embodiment 26. The compound or pharmaceutical composition according to any one of embodiments 1-25, wherein the dosage is administered for at least 24 weeks.
Embodiment 27. The compound or pharmaceutical composition according to any one of embodiments 1-26, wherein the dosage is administered for at least 42 weeks.
Embodiment 28. The compound or pharmaceutical composition according to any one of embodiments 1-27, wherein the dosage is administered for at least 52 weeks.
Embodiment 29. A combined preparation comprising (a) the compound according to any one of embodiments 1-9 or the pharmaceutical composition according to embodiment 10 and (b) at least one second active agent.
Embodiment 30. The combined preparation according to embodiment 29, wherein the second active agent is selected from the group consisting of a vitamin, calcimimetic, bisphosphonate, phosphate binder, thiosulfate, pyrophosphate, citrate, diuretic, antihypertensive, anticholesteraemic agent, phosphodiesterase inhibitor, and combinations thereof.
Embodiment 31. The combined preparation according to embodiment 30, wherein the vitamin is vitamin B, vitamin D, vitamin K, or a combination thereof.
Embodiment 32. The combined preparation according to embodiment 30, wherein the calcimimetic is cinacalcet ((R)-N-[1-(1-naphthyl)ethyl]-3-[3-(trifluoromethyl)phenyl]propan-1-amine, KAI-4169 (etelcalcetide), NPS R-467 (((R)-N-(3-phenylpropyl)-1-(3-methoxyphenyl)ethylamine)), NPS R-568 ((R)-2-chloro-N-(1-(3-methoxyphenyl)ethyl)benzenepropanamine), or a combination thereof.
Embodiment 33. The combined preparation according to embodiment 30, wherein the phosphate binder is a calcium salt, iron salt, lanthanum salt, aluminum salt, magnesium salt, sevelamer salt, or a combination thereof.
Embodiment 34. The combined preparation according to embodiment 30, wherein the bisphosphonate is etidronate, alendronate, risedronate, zoledronate, tiludronate, pamidronate, monidronate, neridronate, parnidronate, olpadronate, clodronate, ibandronate, or a combination thereof.
Embodiment 35. The combined preparation according to embodiment 30, wherein the phosphodiesterase inhibitor is cilostazol, pentoxifylline, or a combination thereof.
Embodiment 36. The combined preparation according to any one of embodiments 29-35, wherein (a) the compound according to any one of embodiments 1-9 or the pharmaceutical composition according to embodiment 10 and (b) the second active agent are administered separately, simultaneously or sequentially.

This invention is further illustrated by the following examples which should not be construed as limiting. The contents of all documents cited throughout this application are incorporated herein in their entirety by reference.

### Example 1

### Slowing Progression of Cardiovascular Calcification with SNF472 in Patients on Hemodialysis: Results of a Randomized, Phase 2b Study

The high cardiovascular morbidity and mortality in patients with end-stage kidney disease (ESKD) could be partially due to extensive cardiovascular calcification (CVC). SNF472, intravenous myo-inositol hexaphosphate, selectively inhibits formation and growth of hydroxyapatite.

In this double-blind, placebo-controlled phase 2b trial compared progression of coronary artery calcium (CAC) volume score and other measurements of CVC by CT scan during 52 weeks of treatment with SNF472 or placebo, in addition to standard therapy, in adult patients with ESKD receiving hemodialysis. Patients were randomized 1:1:1 to SNF472 300 mg (n=92), SNF472 600 mg (n=91), or placebo (n=91) by infusion in the hemodialysis lines thrice weekly during hemodialysis sessions. The primary end point was change in log CAC volume score from baseline to week 52. The primary efficacy analysis combined the SNF472 treatment groups and included all patients who received at least one dose of SNF472 or placebo and had one post-baseline evaluable CT scan by modified intention to treat (mITT) population. In the primary analysis missing data with imputed by Last Observation Carried Forward (LOCF). The per-protocol analysis included all patients who had a week 52 evaluable scan and received 80% exposure to study drug. The mean change in log CAC volume score was 11% (95% CI, 7%-15%) for the combined SNF472 dose group and 20% (95% CI, 14%-26%) for placebo (P=0.016) in the primary analysis (mITT LOCF). The mean change in log CAC volume score was 8% (95% CI, 4%-12%) for the combined SNF472 dose group and 24% (95% CI, 16-32%) for placebo (P=0.001) in a primary per-protocol (PP) analysis.

SNF472 compared to placebo (mITT LOCF) attenuated progression of calcium volume score in the aortic valve significantly (14% [95% CI, 5%-24%] vs 98% [95% CI, 77%-123%], P<0.001). The median change from baseline in thoracic aorta volume score was 234 for placebo and 125 for combined SNF472 dose group.

Death occurred in 7 patients (4%) who received SNF472 and 5 patients (6%) who received placebo. At least one treatment-emergent adverse event occurred in 86%, 92%, and 87% of patients treated with SNF472 300 mg, SNF472 600 mg and placebo, respectively. Most adverse events were mild. Adverse events resulted in discontinuation of SNF472 300 mg, SNF472 600 mg, and placebo for 14%, 29%, and 20% of patients, respectively.

Thus, compared with placebo, SNF472 significantly attenuated progression of CAC and aortic valve calcification in patients with ESKD receiving hemodialysis in addition to standard care.

### Materials and methods

### Study design and participants

A multinational, randomized, placebo-controlled, double-blind Phase 2b trial was conducted among adult patients (18 to 80 years of age) receiving hemodialysis for ≥6 months who had a coronary artery calcium (CAC) Agatston score between 100 and 3500 units at trial entry, as measured on a non-contrast multi-detector CT scanner (MDCT). Calcium Agatston scores were used for study eligibility because they are commonly used for risk stratification in clinical practice. Younger patients (18 to 54 years of age) were also required to have a history of diabetes mellitus (either type 1 or type 2). Key exclusion criteria were scheduled kidney transplant, weight above 136 kg, recent (within 3 months) hospitalization for cardiovascular events (unstable angina, myocardial infarction, stroke, transient ischemic attack, amputation, peripheral or coronary bypass surgery, or unstable heart failure), hypocalcemia (serum calcium <8.0 mg/dL [2.0 mmol/L]), extreme elevation in serum phosphate (>10 mg/dL [3.23 mmol/L] within the last 2 months), uncontrolled hypertension (2 or more consecutive post-dialysis diastolic blood pressure values >100 mmHg within the last 2 months), or expected survival <2 years per investigator.

After screening, eligible patients were randomized 1:1:1 to receive SNF472 300 mg, SNF472 600 mg, or placebo 3 times weekly, infused over 2.5 ± 0.5 hours during each hemodialysis session for 52 weeks.

To maintain blinding, randomization was performed using a centralized electronic randomization system and the study drug was packaged in identical vials containing either SNF472 or physiologic saline. Randomization was stratified by baseline CAC Agatston score (100 to <399, 400 to 1000, or >1000 U). Investigators managed blood pressure, calcium, phosphate, parathyroid hormone, lipids, and anemia according to current guidelines or standard practices at their centers.

At screening and week 52 (or at the time of early discontinuation), imaging of the coronary arteries, aortic valve, and thoracic aorta was performed using MDCT, with a minimum of 64 slices. A central expert who was blinded to the patient's assigned treatment group reviewed each scan and quantified each calcified area using both the volume and Agatston score (Callister T, et al., Radiology 1998; 208:807-814 and Agatston A, et al., J Am Coll Cardiol 1990; 15:827-832). A post-randomization scan was considered evaluable if the expert image reviewer considered it of sufficient quality to be compared to the baseline scan and could calculate a CAV volume score without being hampered by artifact. Patients who discontinued prior to week 52 without withdrawal of consent were asked to undergo CT scanning for assessment of efficacy to pursue maximal available follow-up and minimize missing data.

### End points

The primary efficacy end point was the change from baseline to week 52 in log CAC volume score, which has a known lower variability than the Agatston score (Callister, 2008, *supra*). The primary efficacy analysis combined the two SNF472 treatment groups compared with placebo. Secondary efficacy end points include change from baseline to week 52 for log CAC Agatston score, and changes from baseline to week 52 for log calcium volume score and log calcium Agatston score at the aortic valve and thoracic aorta level, and the proportion of patients with <15% progression in CAC Agatston score at week 52 (Budoff M, et al., JACC Cardiovasc Imaging 2010; 3:1229-1236). Each end point was analyzed for the combined SNF472 dose groups versus placebo and for each SNF472 dose group versus placebo. Other end points included a composite safety end point for cardiovascular outcomes (death from cardiovascular causes, nonfatal myocardial infarction, nonfatal stroke, or heart failure), all-cause mortality, incidence of adverse events (overall, serious, or leading to study drug discontinuation), and changes from baseline to week 52 for clinical laboratory tests.

### Statistical analysis

Analyses were conducted using SAS software version 9.4 (SAS Institute Inc., Cary, NC, USA). The safety population included all randomized patients who received at least one dose of SNF472 or placebo. The modified intention-to-treat analysis population (mITT), which was used for efficacy analyses, consisted of all randomized patients who received at least one dose of SNF472 or placebo and a post-randomization CT scan with a CAC volume score. For the primary analyses of calcification progression at week 52, the last observation (the post-randomization scan) carried forward (LOCF) in the mITT population was used.

The primary efficacy analysis model was an analysis of covariance (ANCOVA) with the change in log score (log[week 52] - log[baseline]) as the dependent variable, and a fixed effect term for randomized treatment group as well as log(baseline) as covariates; the model was stratified by baseline CAC Agatston score. The primary comparison of interest was the SNF472 combined dosing groups versus placebo; supportive comparisons examined differences between each dose and placebo.

Geometric least square (LS) means and 95% confidence intervals (CI) were estimated and back-transformed prior to presentation to provide the mean percent change from baseline to week 52. For each comparison between SNF472 and placebo, geometric LS mean and 95% CI for treatment differences were calculated. Two-tailed p-values <0.05 were considered as statistically significant. For secondary efficacy end points, we used similar ANCOVA models were used for comparing NF472 combined dosing groups versus placebo and SNF472 300 mg and 600 mg doses versus placebo individually. Zero calcium scores for aortic valve and thoracic aorta were imputed with the next smallest value in the relevant treatment arm.

For the primary analyses of calcification progression at week 52, the last observation carried forward (LOCF) was used. In subsequent sensitivity analyses, multiple imputations were implemented to explore the effect of missing data for patients who discontinued the study early. If a patient had only a screening CAC volume score, no imputation was performed. In the first multiple imputation, missing data in each group using distribution implied by the non-missing patient data for that group was used. In the second multiple imputation, missing data for all groups using distribution implied by the non-missing patient data in the placebo arm was imputed.

For the composite safety end point, proportional hazards (Cox) regression to compare SNF472 to placebo, stratified by baseline CAC Agatston score was used.

The planned sample size of 270 patients provided 80% power to test the hypothesis that the log-transformed true difference in progression between the SNF472 combined dosing groups and the placebo group was 0.130, corresponding to a true ratio of 1.139, or 18.5% progression for SNF472 combined dosing groups and 35% progression for the placebo group.

### Results

### Enrollment and disposition

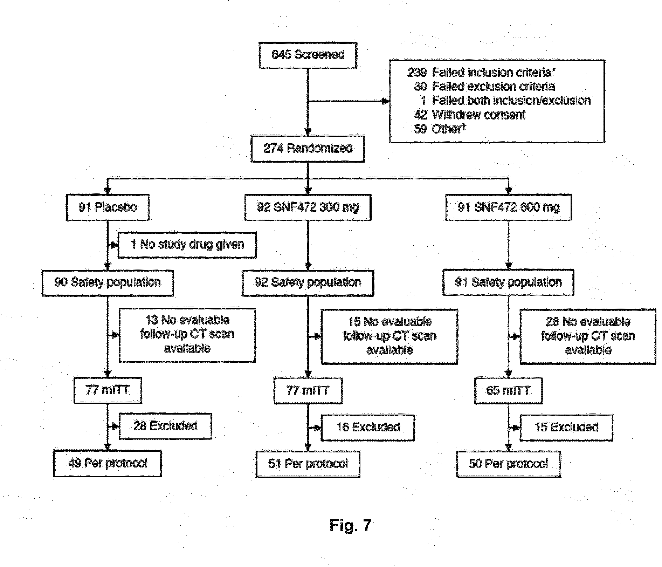

Of 645 patients from 65 centers who were screened for enrollment, 274 patients were randomized to one of the three treatment groups: SNF472 300 mg (n=92), SNF472 600 mg (n=91), or placebo (n=91). Reasons for study discontinuation before week 52 were similar across the treatment groups. Safety evaluations included 92, 91, and 90 patients, respectively, in the SNF472 300 mg, SNF472 600 mg, and placebo groups who received at least one dose of study drug **(****Fig. 7****).** Follow-up CT scans were available for evaluation of calcium volume score progression in the mITT population for 142 patients in the SNF472 combined dosing groups and 77 patients in the placebo group.

### Baseline characteristics

After the randomization code were unblinded for this analysis, patient demographics and clinical characteristics at baseline were shown to be similar across the three treatment groups. At baseline, geometric mean scores in the SNF472 300 mg, SNF472 600 mg, and placebo groups, respectively, were 621, 636, and 634 units for CAC Agatston scores, and 573, 583, and 584 for CAC volume scores. Baseline calcium scores for the coronary arteries and aortic valve are summarized by treatment group in Table 1.

**Table 1**

| Baseline Calcium Scores (Volume and Agatston) | | | |
|---|---|---|---|
| | Placebo (n=77) | SNF472 300 mg (n=77) | SNF472 600 mg (n=65) |
| Coronary artery calcium score | | | |
| Volume | 584±0.88 | 573±0.83 | 583±0.89 |
| Agatston score, units | 634±0.96 | 621±0.91 | 636±0.98 |

| Aortic valve calcium score | | | |
|---|---|---|---|
| Volume | 15.3±1.94 | 27.9±2.04 | 15.5±1.90 |
| Agatston score, units | 8.1±2.47 | 17.5±2.56 | 8.5±2.44 |

| Thoracic aorta calcium score | | | |
|---|---|---|---|
| Volume | 1138±1.82 | 1095±1.87 | 981±1.91 |
| Agatston score, units | 1276±2.00 | 1210±2.08 | 1096±2.14 |

Data are expressed as geometric mean±log(SD) for the modified intention-to-treat population.

### Coronary artery

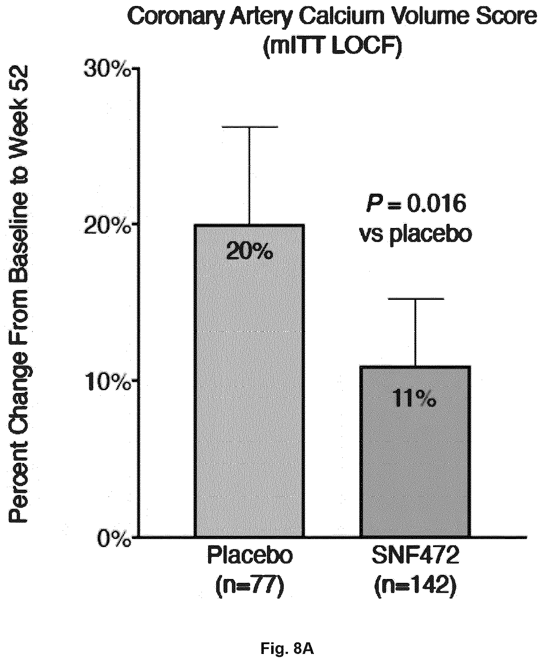

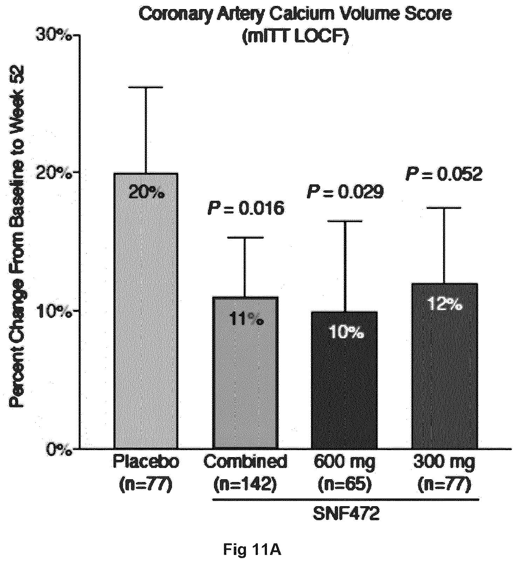

For the primary end point, SNF472 (combined dosing groups) significantly attenuated the progression of CAC compared with placebo: the mean change in CAC volume score from baseline to week 52 was 11% (95% CI, 7%-15%) and 20% (95% CI, 14%-26%), respectively (P=0.016; **Fig. 8A****).** The mean change from baseline to week 52 in CAC volume score was 12% (95% CI, 6%-18%) for the 300 mg dose group (P=0.052 vs placebo) and 10% (95% CI, 4%-17%) for the 600 mg dose group (P=0.029 vs placebo) **(****Fig. 11A****).**

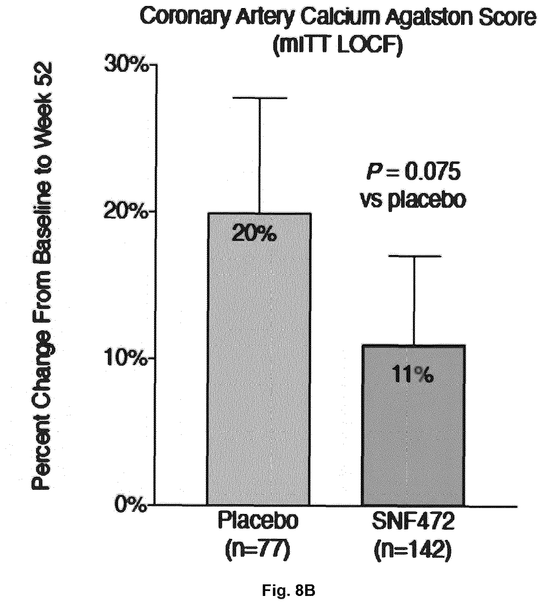

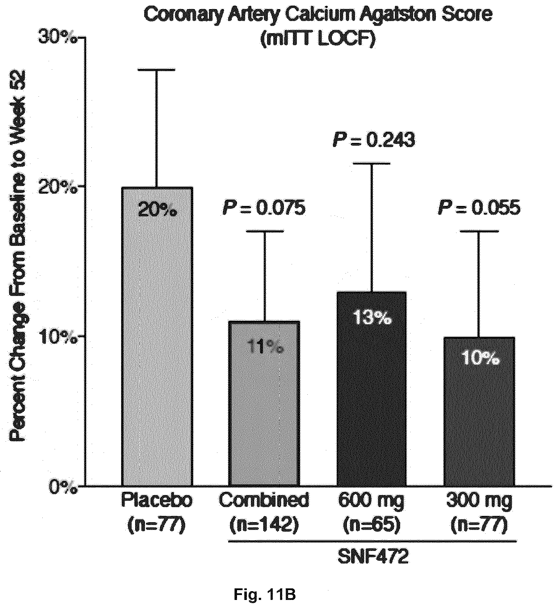

Corresponding results using the CAC Agatston score were a mean change of 11% (95% CI, 6%-17%) for SNF472 combined dosing groups and 20% (95% CI, 12%-28%) for placebo (P=0.075; **Fig. 8B****).** The mean change in CAC Agatston score was 10% (95% CI, 3%-17%) for the 300 mg dose group (P=0.055 vs placebo) and 13% (95% CI, 6%-22%) for the 600 mg dose group (P=0.24 vs placebo) **(****Fig. 11B**).

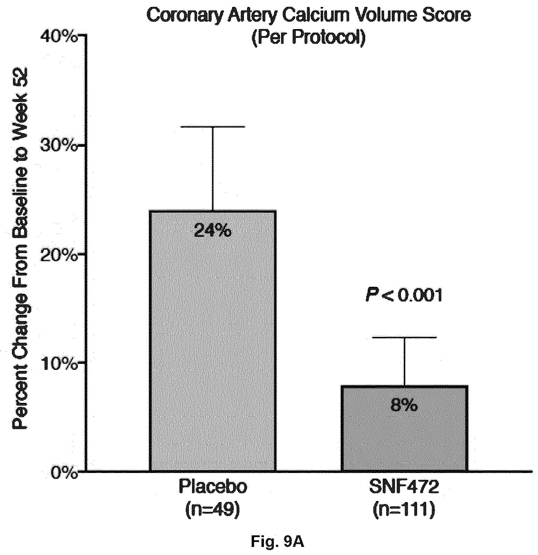

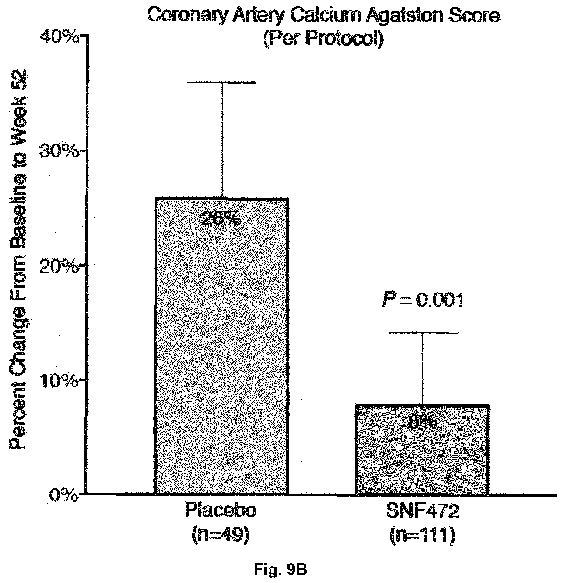

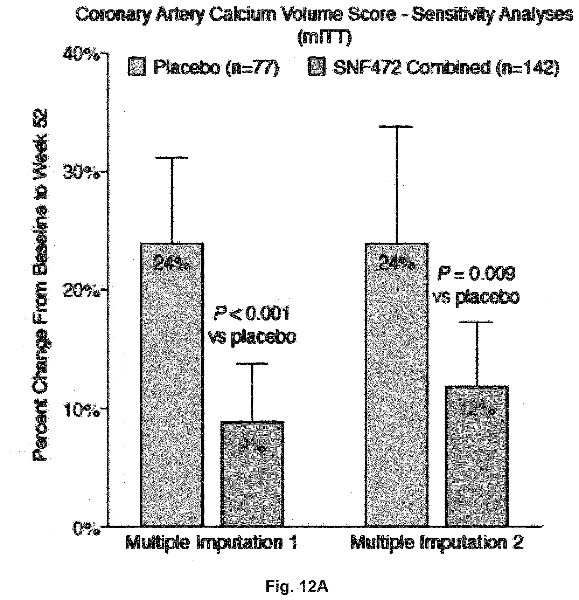

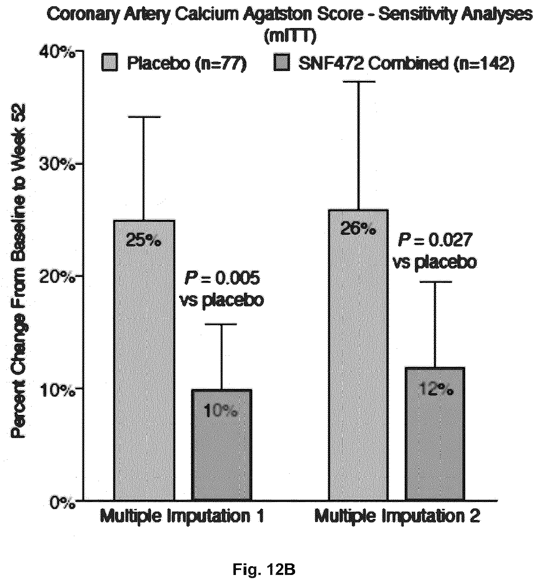

Somehow better results for progression of calcium volume and Agatston scores were obtained in the per-protocol population of patients that completed 52 weeks of treatment **(****Fig. 9A** and **9B****)** and in sensitivity analyses that used multiple imputation for missing data in the mITT population **(****Fig. 12A** and **B****).**

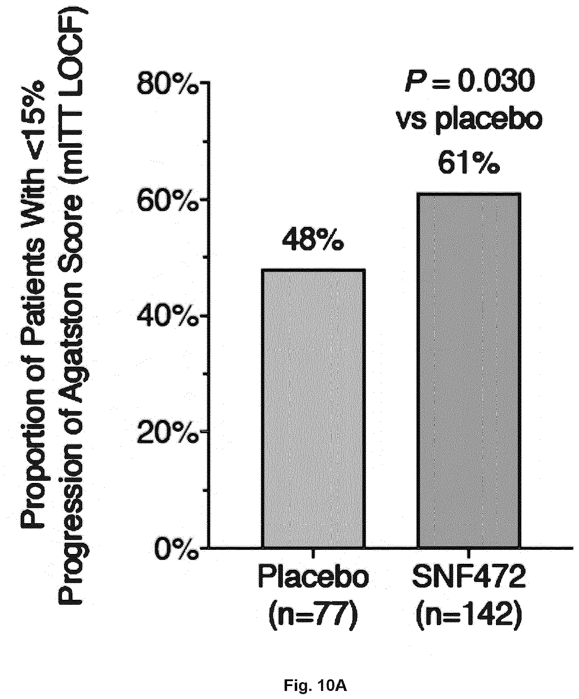

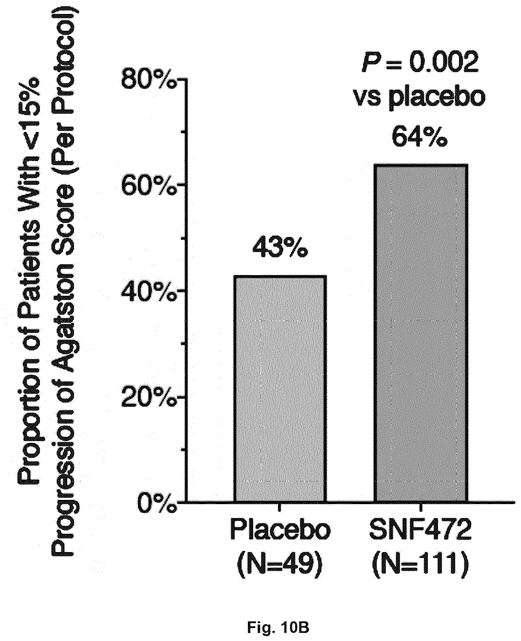

The proportion of patients with <15% progression in CAC Agatston score at week 52 was 61% for SNF472 combined dosing groups and 48% for placebo in the mITT population (P=0.030; **Fig. 10A****)** and 64% and 43%, respectively, in the per-protocol population (P=0.002; **Fig. 10B****).**

### Aortic valve

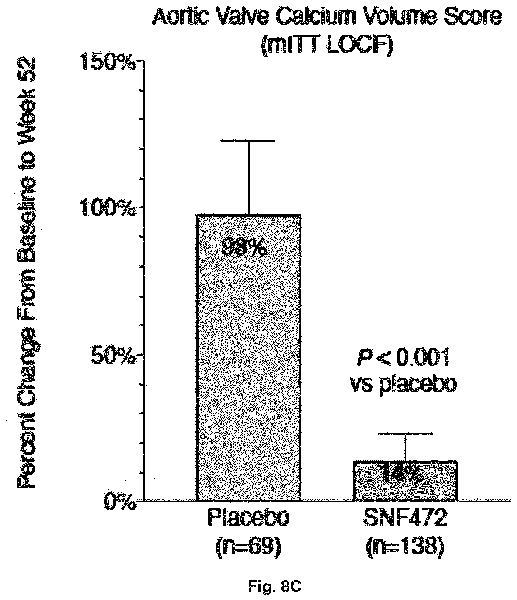

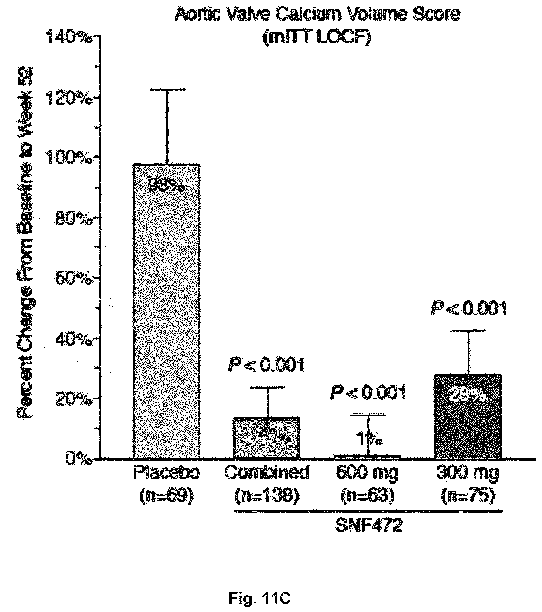

Changes from baseline to week 52 in calcium volume scores in the aortic valve were 14% (95% CI, 5%-24%) for SNF472 combined dosing groups and 98% (95% CI, 77%-123%) for placebo (P<0.001; **Fig. 8C****).** The mean change from baseline to week 52 was 28% (95% CI, 14%-43%) for the 300 mg dose group (P<0.001 vs placebo) and 1% (95% CI, -10%-14%) for the 600 mg dose group (P<0.001 vs placebo) **(****Fig. 11C****).**

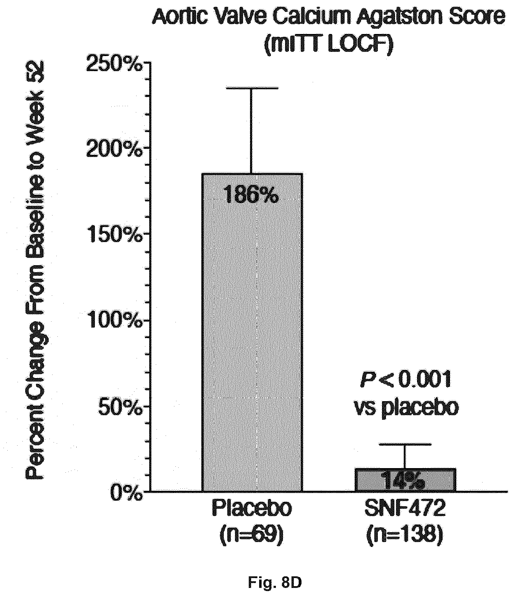

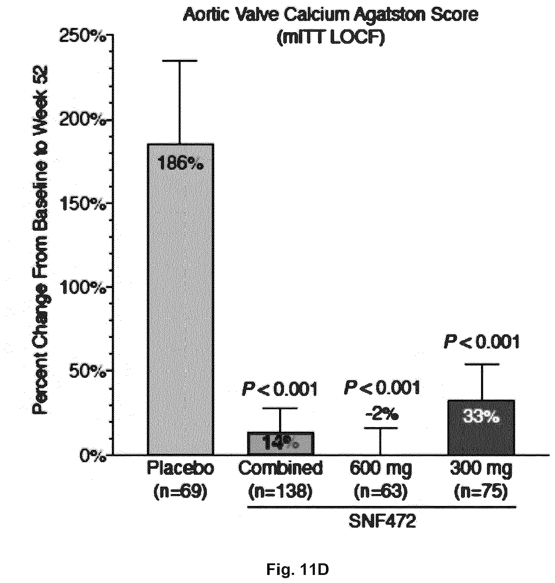

Corresponding results for calcium Agatston scores were 14% (95% CI, 2%-28%) for SNF472 combined dosing groups and 186% (95% CI, 145%-235%) for placebo (P<0.001; **Fig. 8D****).** The mean change from baseline to week 52 was 33% (95% CI, 14%-54%) for the 300 mg dose group (P<0.001 vs placebo) and -2% (95% CI, -16%-16%) for the 600 mg dose group (P<0.001 vs placebo) **(****Fig. 11D**).

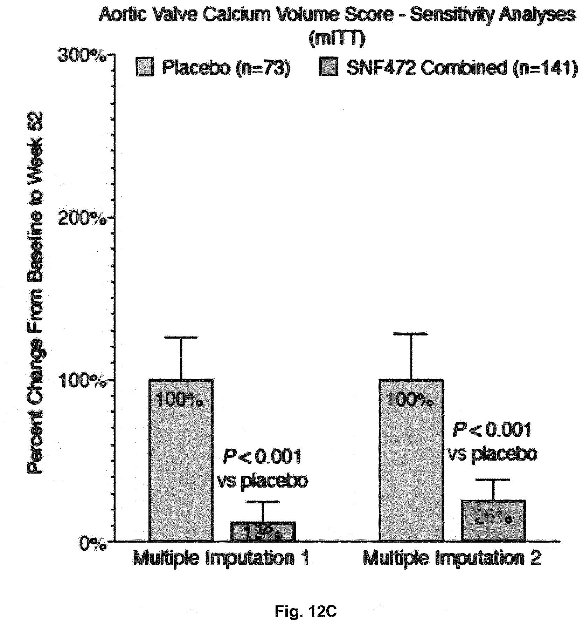

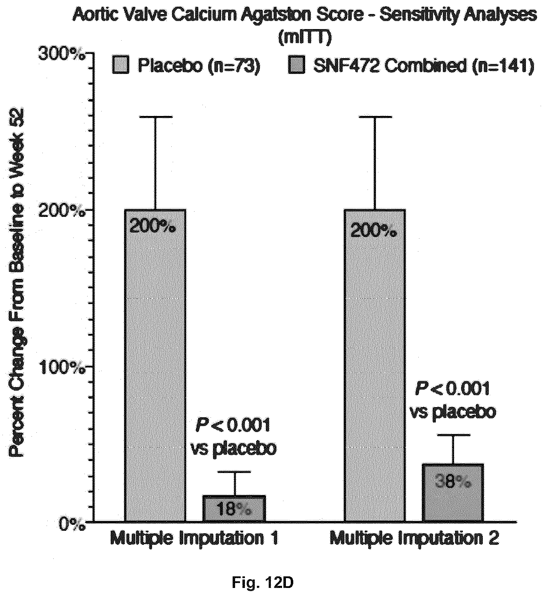

Similar results for progression of calcium volume and Agatston scores were obtained in sensitivity analyses that used multiple imputation for missing data in the mITT population **(****Fig. 12C** and **12D****).**

### Thoracic aorta

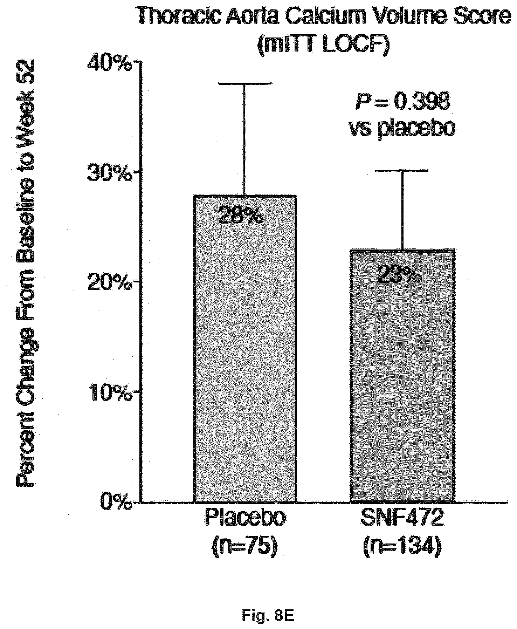

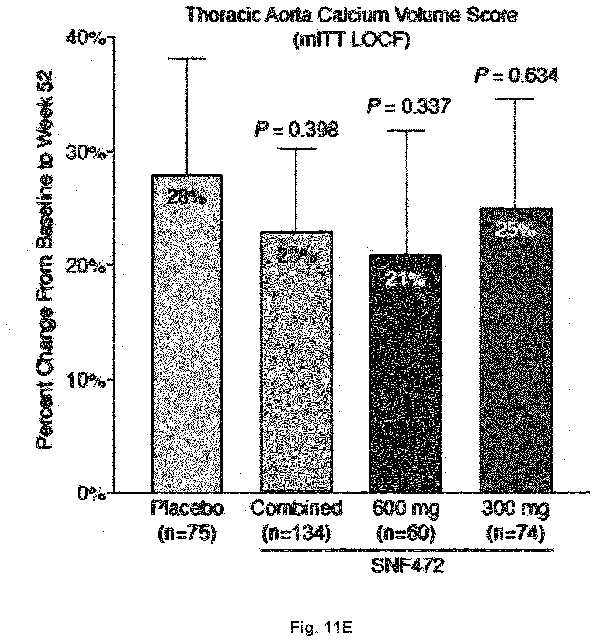

Changes from baseline to week 52 in calcium volume scores in the thoracic aorta were 23% (95% CI, 16%-30%) for SNF472 combined dosing groups and 28% (95% CI, 19%-38%) for placebo (P=0.40; **Fig. 8E****).** The mean change from baseline to week 52 was 25% (95% CI, 16%-35%) for the 300 mg dose group (P=0.63 vs placebo) and 21% (95% CI, 11%-32%) for the 600 mg dose group (P=0.34 vs placebo) **(****Fig. 11E****).**

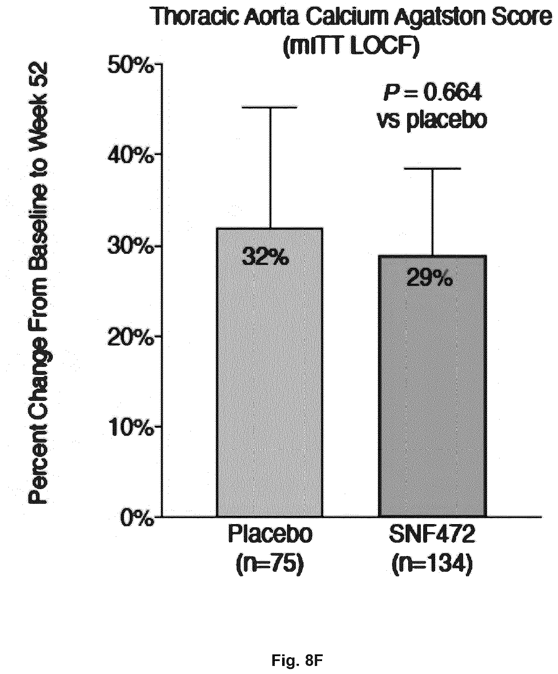

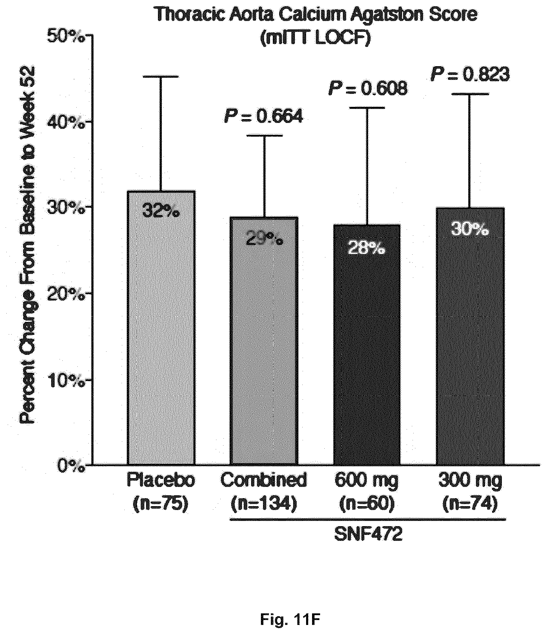

Corresponding results for calcium Agatston scores were 29% (95% CI, 20%-38%) for SNF472 combined dosing groups and 32% (95% CI, 21%-45%) for placebo (P=0.66; **Fig. 8F****).** The mean change from baseline to week 52 was 30% (95% CI, 19%-43%) for the 300 mg dose group (P=0.82 vs placebo) and 28% (95% CI, 15%-42%) for the 600 mg dose group (P=0.61 vs placebo) **(****Fig. 11F****).**

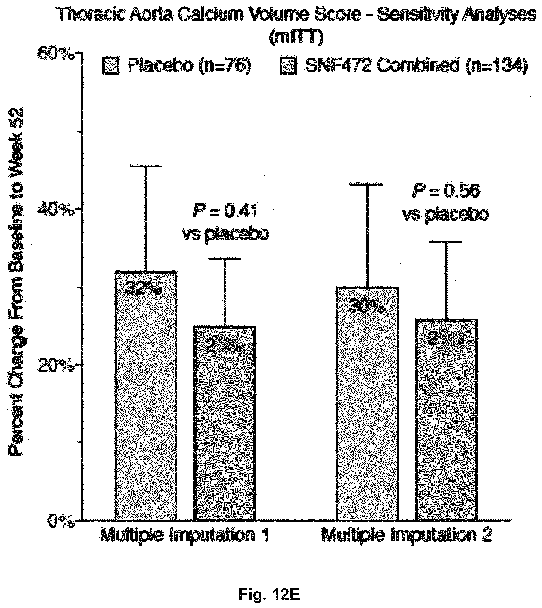

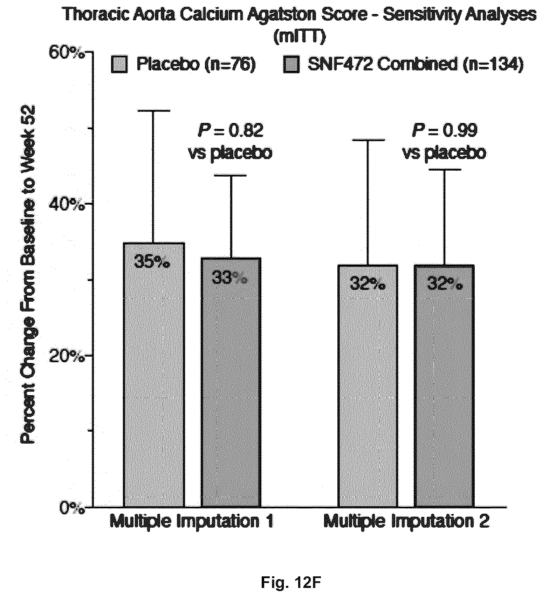

Similar results for progression of calcium volume and Agatston scores were obtained in sensitivity analyses that used multiple imputation for missing data in the mITT population **(****Fig. 12E** and **12F****).**

The median change from baseline in thoracic aorta volume score was 204 for placebo and 131 for combined SNF472 dose group (mITT LOCF), which represents a 35.8% difference. The median change from baseline in thoracic aorta volume score was 234 for placebo and 125 for combined SNF472 dose group (PP), which represents a 46.6% difference.

### Composite safety end point

The incidence of the composite safety end point in the SNF472 300 mg, SNF472 600 mg, and placebo groups, respectively, was 8%, 7%, and 11% **(Table 2).** The hazard ratio for the composite safety end point for SNF472 combined dosing groups vs placebo was 0.60 (95% CI, 0.26-1.37; P=0.22).

**Table 2**

| Composite Safety End Point | | | |
|---|---|---|---|
| | Placebo (n=90) | SNF472 300 mg (n=92) | SNF472 600 mg (n=91) |
| Any composite safety end point event | 10 (11.1) | 7 (7.6) | 6 (6.6) |
| Cardiovascular death | 0 | 0 | 1 (1.1) |
| Nonfatal myocardial infarction | 4 (4.4) | 4 (4.3) | 1 (1.1) |
| Nonfatal stroke | 2 (2.2) | 1 (1.1) | 0 |
| Heart failure | 4 (4.4) | 1 (1.1) | 2 (2.2) |
| Nonfatal cardiac arrest | 0 | 1 (1.1) | 2 (2.2) |

Data are expressed as number (%).

### Adverse events

Adverse events occurred in 79 of 92 patients (86%) in the SNF472 300 mg group, 84 of 91 patients (92%) in the SNF472 600 mg group, and 78 of 90 patients (87%) in the placebo group. Serious adverse events occurred in 38 patients (41%) in the SNF472 300 mg group, 55 patients (60%) in the SNF472 600 mg group, and 49 patients (54%) in the placebo group.

Adverse events led to study drug withdrawal for 13 patients (14%) in the SNF472 300 mg group, 26 patients (29%) in the SNF472 600 mg group, and 18 patients (20%) in the placebo group. A majority of these discontinuations were for kidney transplant in 27 patients overall (6% SNF472 300 mg, 12% SNF472 600 mg, and 9% placebo). No other event led to study drug withdrawal for more than two patients.

No clinically significant abnormalities were identified from analyses of clinical laboratory values (hematology and chemistry) with SNF472 compared with placebo.

Death occurred in one patient in the SNF472 300 mg group (multiple organ dysfunction syndrome), 6 patients in the SNF472 600 mg group (one each due to aortic stenosis, arteriosclerosis, hypotension, cardiac arrest, septic shock, and epilepsy), and five patients in the placebo group (one each due to cardiac failure congestive, multiple organ dysfunction syndrome, sepsis, post-procedural complication, and renal transplant). None of the deaths were considered to be related to the study drug.

### Discussion

This international, multicenter, randomized, double-blind placebo-controlled clinical trial, showed that SNF472, a selective inhibitor of the formation and growth of hydroxyapatite crystals, when infused during each hemodialysis session for one year, attenuated the progression of coronary artery and aortic valve calcification relative to placebo. These results are surprising in view of the concomitant use of drugs shown in prior studies to potentially attenuate CAC such as the non-calcium-based phosphate binder sevelamer and the calcimimetic cinacalcet (Raggi P, et al., Nephrol Dial Transplant. 2011; 26:1327-1339, Chertow G, et al., Kidney Int. 2002; 62:245-2352, and Sadek T, et al., Nephrol Dial Transplant. 2003; 18:582-588). In those prior studies, patients receiving hemodialysis assigned placebo demonstrated a 35-40% average rate of progression of CAC per year, nearly double the rate of progression seen in placebo-treated patients in this study. Of interest, the study power calculations assumed a CAC volume score progression of 18.5% for SNF472-treated patients and 35% for placebo-treated patients (a 47% relative difference in progression). These assumptions were based on prior published rates of progression. While the observed progression of CAC in placebo-treated patients in this study was slower than expected, in part due to the use of non-calcium-based phosphate binders and calcimimetics in many patients, the relative difference of 45% was very close to the study initial expectations.

At baseline, all patients had coronary calcium with CAC Agatston scores between 100 and 3500 units. Additionally, 57% of patients had calcification of the aortic valve (Bellasi, 2019, *supra*)*.* Since the primary aim of the study was to show that SNF472 effectively inhibits progression of coronary calcification, patients with moderate to high CAC scores were enrolled, as they generally exhibit a faster progression of calcification.

Previous meta-analyses showed that the prescription of non-calcium-based phosphate binders is associated with attenuation of the progression of cardiovascular calcification as well as improved survival (Jamal S, et al., Lancet 2013; 382:1268-1277 and Patel L, et al., Clin J Am Soc Nephrol. 2016; 11:232-244). The association between cardiovascular calcification and morbidity and mortality has been shown in the general population in numerous prior publications. As a result, CAC has been recognized as a risk-modifying marker in the most recent guidelines from the American Heart Association and American College of Cardiology for primary prevention of atherosclerotic cardiovascular disease. Moreover, both CAC measured on CT and simple measures of calcification such as radial and femoral artery as well as aortic calcification seen on planar X-rays have been shown to be strongly associated with adverse events in ESKD (Blacher J, et al., Hypertension 2001; 38:938-942, Adragao T, et al., Nephrol Dial Transplant. 2009; 24:997-1002, Disthabanchong S, et al., Int Urol Nephrol. 2018; 50:355-364, Kwon H, et al., Kidney Res Clin Pract. 2014; 33:95-102, Okuno S, et al., Am J Kidney Dis. 2007; 49:417-425, and Verbeke F, et al., Clin J Am Soc Nephrol. 2011; 6:153-159).

It is well established that patients with ESKD develop cardiovascular calcifications well in excess of patients with normal or near normal kidney function, and that there are two distinct sites of arterial calcification in these patients: intimal and medial. The former is linked with traditional risk factors for atherosclerosis and inflammation, while the latter is found mostly (if not exclusively) in arteries of medium and large caliber and is more closely associated with the mineral and bone disorder typical of ESKD. Calcification of medium and large arterial conduits is responsible for reduced vascular compliance that can induce left ventricular hypertrophy and systolic/diastolic dysfunction in the long-term. Reduced compliance, as measured by increased pulse pressure velocity, can result in reduced diastolic filling of the coronary arteries with a state of relative ischemia of the myocardium even in the absence of obstructive luminal disease. Hence, arterial calcification in ESKD should be interpreted as a harbinger of severe complications and therefore worth pursuing as a target of therapy. Equally important is the impact of valvular calcification in ESKD, particularly calcification of the aortic valve (Marwick T, et al., Kidney Int. 2019; 96:836-849). Numerous patients suffer from severe calcification of the left sided cardiac valves with subsequent regurgitation or restriction of the mobility of the valve leaflets. These conditions in turn are associated with progressive left ventricular fibrosis, left ventricular hypertrophy and dysfunction as well as endocarditis and severe conduction abnormalities. Additionally, surgical and interventional treatments proven efficacious in the general population may not exert a similar benefit in patients with ESKD. The significant reduction in progression of calcification of the aortic valve seen in this study is therefore encouraging.

Independent of the pathophysiology of cardiovascular calcification, the final step entails the formation of crystals of hydroxyapatite through active processes mimicking bone assembly. In this regard, SNF472 is a specific inhibitor of hydroxyapatite crystallization and is effective at delaying the progression of coronary artery and aortic valve calcification.

### Conclusions

Compared with placebo, 52 weeks of treatment with SNF472 significantly attenuated progression of coronary artery and aortic valve calcification in patients with ESKD receiving hemodialysis. These results were obtained on a background of frequent utilization of drugs with the ability to attenuate, at least partially, the progression of cardiovascular calcification in patients with ESKD.

## Claims

1. A compound of general formula I, or a pharmaceutically acceptable salt thereof: 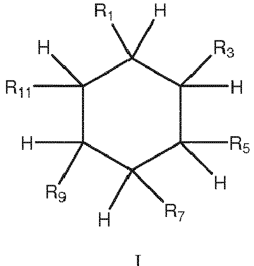 wherein:
(i) R₁, R₃, R₅, R₇, R₉, and R₁₁ are independently selected from OH, a radical of formula II, III, IV and a heterologous moiety: 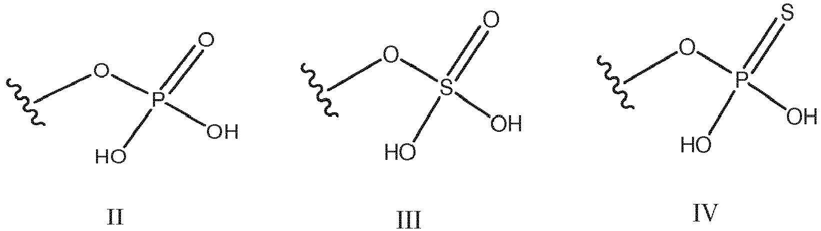
(ii) at least one of R₁, R₃, R₅, R₇, R₉, and R₁₁ is selected from a radical of formula II, III and IV; and
(iii) zero, one, two or three of R₁, R₃, R₅, R₇, R₉, and R₁₁ is a heterologous moiety;
for use in the treatment, inhibition of progression, or prevention of cardiovascular calcification, or a disease, condition or symptom associated with cardiovascular calcification in a subject in need thereof, wherein (a) the compound is in a form suitable for parenteral, topical, or enteral administration, and (b) the compound is administered to the subject in a non-bolus prolonged release form in an effective dosage of about 200 mg to about 700 mg per administration.

2. The compound according to claim 1, wherein the heterologous moiety is selected from a radical of formula V, a radical of formula VI and a radical of formula VII: 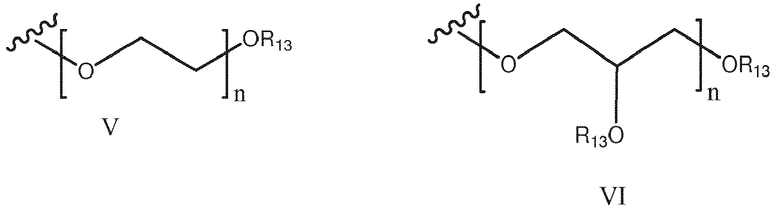 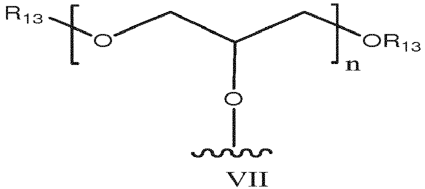 wherein:
n is an integer in the range from 2 to 200, and
R₁₃ is selected from H, methyl, and ethyl.

3. The compound according to any one of claims 1-2, wherein the cardiovascular calcification is coronary artery calcification, aortic artery calcification, or aortic valve calcification.

4. The compound according to any one of claims 1-3, wherein the compound of formula I is inositol hexaphosphate.

5. The compound according to claim 4, wherein the inositol hexaphosphate is myo-inositol hexaphosphate.

6. The compound according to any one of claims 4-5, wherein the inositol hexaphosphate is in its hexasodium salt form.

7. The compound according to any one of claims 1-3, wherein one or two of R₁, R₃, R₅, R₇, R₉, and R₁₁ is selected from a radical of formula V, VI and VII.

8. The compound according to claim 7, wherein R₁, R₅, R₉, and R₁₁ are a radical of formula II and R₃ and R₇ are a radical of formula V.

9. The compound according to claim 8, wherein the radical of formula V has n in the range from 2 to 200 and R₁₃ is H.

10. A pharmaceutical composition for use in the treatment, inhibition of progression, or prevention of cardiovascular calcification in a subject in need thereof comprising the compound according to any one of claims 1-9 and pharmaceutically acceptable excipients and carriers.

11. The compound according to any one of claims 1-9 or the pharmaceutical composition according to claim 10, wherein the subject is with kidney failure.

12. The compound according to any one of claims 1-9 or the pharmaceutical composition according to claim 10, wherein the subject is on dialysis.

13. The compound or pharmaceutical composition according to any one of claims 1-12, wherein the parenteral administration is intravenous, subcutaneous, intramuscular or by intravenous infusion.

14. The compound or pharmaceutical composition according to claim 13, wherein the intravenous infusion is administered using a dialysis apparatus.

15. The compound or pharmaceutical composition according to any one of claims 1-14, wherein the subject is human.

16. The compound or pharmaceutical composition according to any one of claims 1-15, wherein the compound is administered to the subject in a dosage of about 250 mg to about 650 mg per administration to the subject.

17. The compound or pharmaceutical composition according to any one of claims 1-16, wherein compound is administered to the subject in a dosage of about 300 mg to about 600 mg per administration to the subject.

18. The compound or pharmaceutical composition according to any one of claims 1-17, wherein compound is administered to the subject in a dosage of about 350 mg to 550 mg per administration to the subject.

19. The compound or pharmaceutical composition according to any one of claims 1-18, wherein the dosage is administered in single daily doses.

20. The compound or pharmaceutical composition according to any one of claims 1-19, wherein the dosage is administered as multiple daily doses.

21. The compound or pharmaceutical composition according to any one of claims 1-20, wherein the dosage is administered at least once a week.

22. The compound or pharmaceutical composition according to any one of claims 1-21, wherein the dosage is administered 2, 3, 4, 5, 6 or 7 times per week.

23. The compound or pharmaceutical composition according to any one of claims 1-22, wherein the dosage is administered for at least one week.

24. The compound or pharmaceutical composition according to any one of claims 1-23, wherein the dosage is administered for about 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, or 52 weeks.

25. A combined preparation comprising (a) the compound according to any one of claims 1-9 or the pharmaceutical composition according to claim 10 and (b) at least one second active agent.
